(19) Europäisches Patentamt
European Patent Office
Office européen des brevets

(11) Veröffentlichungsnummer: **0 289 842 B1**

(12) **EUROPÄISCHE PATENTSCHRIFT**

(45) Veröffentlichungstag der Patentschrift: **27.11.91**

(51) Int. Cl.$^5$: **C07C 271/16**, A01N 47/12, A01N 47/16, A01N 47/18

(21) Anmeldenummer: **88106119.6**

(22) Anmeldetag: **18.04.88**

# (54) Mittel zur Insekten- und Milbenabwehr.

(30) Priorität: **28.04.87 DE 3714058**
**16.01.88 DE 3801082**

(43) Veröffentlichungstag der Anmeldung:
**09.11.88 Patentblatt 88/45**

(45) Bekanntmachung des Hinweises auf die Patenterteilung:
**27.11.91 Patentblatt 91/48**

(84) Benannte Vertragsstaaten:
**AT BE CH DE ES FR GB GR IT LI LU NL SE**

(56) Entgegenhaltungen:
**EP-A- 0 169 169**
**DE-C- 1 150 973**
**US-A- 3 442 889**
**US-A- 4 555 515**

**PATENT ABSTRACTS OF JAPAN, vol. 9, no. 254 (C-308)[1977], 11th October 1985; & JP - A - 60 109 555 (NIHON IYAKUHIN KOGYO K.K.) 15-06-1985**

**CHEMICAL ABSTRACTS, vol. 104, January 1986, pages 512,513, abstract no. 5644p, Columbus, Ohio, US; & JP - A - 60 109 555**

(73) Patentinhaber: **BAYER AG**

**W-5090 Leverkusen 1 Bayerwerk(DE)**

(72) Erfinder: **Krüger, Bernd-Wieland, Dr.**
**Sillerstrasse 49**
**W-5600 Wuppertal 11(DE)**
Erfinder: **Sasse, Klaus, Dr.**
**Puetzweg 13**
**W-5060 Bergisch-Gladbach 2(DE)**
Erfinder: **Hoever, Franz-Peter, Dr.**
**Kunstfelder Strasse 25**
**W-5000 Köln 80(DE)**
Erfinder: **Nentwig, Günther, Dr.**
**Wolfskaul 2**
**W-5000 Köln 80(DE)**
Erfinder: **Behrenz, Wolfgang, Dr.**
**Untergründemich 14**
**W-5063 Overath(DE)**

EP 0 289 842 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von teilweise bekannten substituierten α,ω-Aminoalkohol-Derivaten als insekten- und milbenabwehrende Mittel. Durch die vorliegende Erfindung werden weiterhin neue substituierte α,ω-Aminoalkohol-Derivate bereitgestellt.

Mittel, die Insekten und Milben abweisen (Repellents), haben die Aufgabe, schädliche oder lästige Gliederfüßler von Berührung, sowie vom Stechen und Saugen oder Beißen an für sie anlockenden Oberflächen, etwa der Haut von Tieren und Menschen abzuhalten, wenn diese zuvor mit solchen Mitteln behandelt wurden.

Als Repellents wurden bereits zahlreiche Wirkstoffe vorgeschlagen. (Vergl. z.B. K.H. Büchel in Chemie der Pflanzenschutz- und Schädlingsbekämpfungsmittel; Herausgeber: R. Wegler, Bd. 1, Springer Verlag Berlin, Heidelberg, New York, 1970 S. 487 ff).

Besonders bekannt und seit längerer Zeit in Gebrauch sind 3-Methyl-benzoesäurediethylamid (DEET), Dimethylphthalat und 2-Ethyl-hexandiol-1,3, von denen vor allem das DEET in der Praxis eine erhebliche Bedeutung erlangt hat (siehe z.B. R.K. Kocher, R.S. Dixit, C.I. Somaya; Indian J. Med. Res. 62, 1 (1974)).

Ein erheblicher Nachteil der bekannten Repellents ist ihre zum Teil relativ kurze (nur wenige Stunden) anhaltende Dauerwirkung.

Ein Teil der durch die nachfolgende Formel (I) definierten Verbindungen ist bekannt.

Siehe dazu die Deutsche Auslegeschrift Nr. 1 288 587, Kolumne 2, 1. Formel mit der Bedeutung R = Methyl. Es handelt sich hier um ein Zwischenprodukt für die Herstellung von N-(3-Carbamoyloxyalkyl)-carbamidsäureethylester, welcher als sedierend wirkendes Arzneimittel eingesetzt wird. Siehe dazu auch die veröffentlichte Europäische Patentanmeldung EP 0 144 825 A1, Verbindung Nr. 37 auf Seite 43 der vorgenannten Publikation, welche als Zwischenprodukt für die Herstellung von antibiotisch wirksamen Verbindungen dient.

Aus DE-C 1 150 973 sind ebenfalls einige analog strukturierte Verbindungen bekannt, welche als Arzneinmittel eingesetze werden. Diese Verbindungen werden in dem Ansprüchen durch Disclaimer ausgenomnen.

Eine insekten- und milbenabweisende Wirkung dieser Verbindungen ist jedoch bisher nicht bekannt geworden.

Es wurde nun gefunden, daß die teilweise bekannten substituierten α,ω-Aminoalkohol-Derivate der Formel I

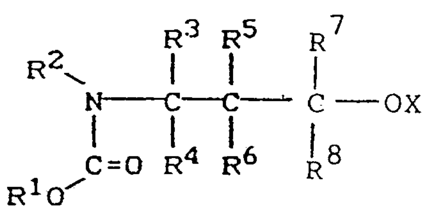

(I)

in welcher

| | |
|---|---|
| X | für Wasserstoff, $COR^{11}$ oder $R^{13}$ steht, |
| $R^1$ | für $C_1$-$C_7$-Alkyl, $C_3$-$C_7$-Alkenyl oder $C_2$-$C_7$-Alkinyl steht, |
| $R^2$,$R^{11}$,$R^{13}$ | gleich oder verschieden sind und für $C_1$-$C_6$-Alkyl oder $C_2$-$C_7$-Alkenyl stehen, |
| $R^3$-$R^8$ | gleich oder verschieden sind und für Wasserstoff oder $C_1$-$C_6$-Alkyl stehen, wobei $R^2$ und $R^3$ oder $R^3$ und $R^7$ oder $R^3$ und $R^5$ oder $R^5$ und $R^7$ gemeinsam mit den Atomen, an welche sie gebunden sind, auch einen 5- oder 6-gliedrigen monocyclischen Ring bilden können, |

eine starke insekten- und milbenabweisende Wirkung (Repellentwirkung) besitzen.

Die Repellentwirkung ist erheblich besser als die der aus dem Stand der Technik bekannten Repellents. Die erfindungsgemäßen Wirkstoffe stellen somit eine wertvolle Bereicherung der Technik dar.

Die vorliegende Erfindung betrifft somit die Verwendung substituierter α,ω-Aminoalkoholderivate der allgemeinen Formel I zur Insekten- und Milbenabwehr.

Weiterhin betrifft die Erfindung insekten- und milbenabweisende Mittel, gekennzeichnet durch den Gehalt an mindestens einem substituierten α,ω-Aminoalkoholderivat der allgemeinen Formel I.

Die erfindungsgemäßen Mittel, die mindestens ein Derivat der Formel I enthalten, können auch weitere Insektenabwehrmittel enthalten. Hier kommen alle praktisch üblichen Repellentien in Frage (vergl. z.B. K.H.

Büchel in Chemie der Pflanzenschutz- und Schädlingsbekämpfungsmittel; Herausg.: R. Wegler, Bd. 1, Springer Verlag Berlin, Heidelberg, New York, 1970, S. 487 ff).

Im Falle der Repellentkombinationen werden bevorzugt die substituierten $\alpha,\omega$-Aminoalkohole der allgemeinen Formel I zusammen mit repellenten Carbonsäureamiden, 1,3-Alkandiolen und Carbonsäureestern verwendet. Im einzelnen seien genannt: 3-Methyl-benzoesäurediethylamid (DEET), 2-Ethyl-hexandiol-1,3 (Rutgers 612) und Phthalsäuredimethylester.

Die erfindungsgemäß verwendbaren substituierten $\alpha,\omega$-Aminoalkoholderivate sind durch die allgemeine Formel (I) charakterisiert.

Besonders bevorzugt werden Verbindungen der allgemeinen Formel (I) als Repellentien verwendet, in welcher

| | |
|---|---|
| X | für Wasserstoff oder $R^{13}$ steht, wobei $R^{13}$ für $C_1$-$C_6$-Alkyl steht, |
| $R^1$ | für $C_1$-$C_7$-Alkyl oder $C_3$-$C_7$-Alkenyl steht, |
| $R^4$ bis $R^8$ | gleich oder verschieden sind und für Wasserstoff oder $C_1$-$C_6$-Alkyl stehen, |
| $R^2$ und $R^3$ | gemeinsam mit den Atomen, an welche sie gebunden sind, einen 5- oder 6-gliedrigen monocyclischen Ring bilden. |

Weiterhin werden Verbindungen bevorzugt, in denen $R^1$ für $C_1$-$C_7$-Alkyl oder $C_3$-$C_7$-Alkenyl steht, X für $COR^{11}$ oder $R^{13}$ steht, $R^2$ und $R^{11}$ gleich oder verschieden sind und für $C_1$-$C_6$-Alkyl stehen, $R^3$ bis $R^8$ gleich oder verschieden sind und für Wasserstoff oder $C_1$-$C_6$-Alkyl stehen, und $R^{13}$ für $C_1$-$C_6$-Alkyl steht.

Ganz besonders bevorzugt werden die Verbindungen der allgemeinen Formel (I) als Repellentien verwendet, in denen $R^1$ für $C_1$-$C_4$-Alkyl steht,

| | |
|---|---|
| $R^2$, $R^{11}$ und $R^{13}$ | gleich oder verschieden sind und für $C_1$-$C_6$-Alkyl stehen, $R^3$ bis $R^8$ für Wasserstoff stehen und X für Wasserstoff, $COR^{11}$ oder $R^{13}$ steht, wobei $R^{11}$ und $R^{13}$ die vorgenannte Bedeutung haben. |

Weiterhin werden ganz besonders bevorzugt Verbindungen der allgemeinen Formel (I) als Repellentien verwendet, in denen $R^1$ für $C_3$-$C_4$-Alkyl steht, $R^2$ und $R^3$ gemeinsam mit den Atomen, an die sie gebunden sind, einen 6-gliedrigen Ring bilden, $R^4$ bis $R^8$ für Wasserstoff steht und X für Wasserstoff und $R^{13}$ steht, wobei $R^{13}$ für $C_1$-$C_4$-Alkyl steht.

Die Verbindungen der allgemeinen Formel (I) sind entweder bekannt oder können nach bekannten Methoden und Verfahren hergestellt werden (vergl. z.B. Cesare Ferri, Reaktionen der organischen Synthese, Georg Thieme Verlag Stuttgart, 1978, S. 223 und S. 450).

Man erhält demgemäß die Verbindungen der Formel (I),

a) wenn man die an sich bekannten oder nach bekannten Verfahren herstellbaren $\alpha,\omega$-Aminoalkohole (vergl. z.B. Cesare Ferri, Reaktionen der org. Synthese, Georg Thieme Verlag Stuttgart, 1978, S. 211 ff bzw. 496-497) der Formel (II)

(II)

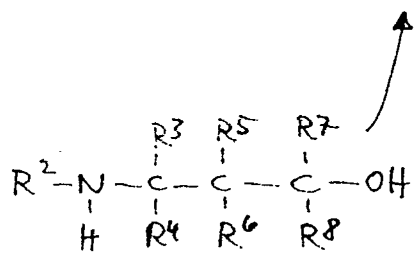

worin $R^2$ bis $R^8$ die unter Formel (I) angegebene Bedeutung haben,

zunächst mit an sich bekannten Chlorkohlensäureestern der Formel (III)

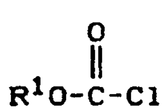

(III),

wobei

$R^1$ die unter Formel (I) angegebene Bedeutung hat,
gegebenenfalls in Gegenwart eines Säureakzeptors, wie z.B. Triethylamin oder Kaliumcarbonat und gegebenenfalls unter Verwendung eines Verdünnungsmittels, wie z.B. Toluol, $CH_2Cl_2$, Tetrahydrofuran oder Acetonitril, bei Temperaturen zwischen -40 und 110 ° C, umsetzt.

Für die Herstellung von Verbindungen der allgemeinen Formel (I), in denen X verschieden von Wasserstoff ist, erfolgt dann in einem zweiten Reaktionsschritt, gegebenenfalls nach Isolierung des Zwischenproduktes mit freier OH-Gruppe, die weitere Acylierung/Alkylierung mit an sich bekannten Carbonsäurechloriden der Formel (IV)

$R^{11}COCl$ (IV),

zur Herstellung von Verbindungen der Formel (I) mit X = $COR^{11}$;
oder mit Alkylhalogeniden der Formel (VI)

$R^{13}$-Y (VI),

zur Herstellung von Verbindungen der Formel (I) mit X = $R^{13}$;
wobei in den Formeln (IV), (VI) Y für Chlor, Brom oder Iod, vorzugsweise für Brom oder Iod steht und $R^{11}$ und $R^{13}$ die obengenannte Bedeutung haben,
gegebenenfalls in Gegenwart eines Säureakzeptors wie z.B. Triethylamin oder Kaliumcarbonat oder einer Base wie Natriumhydrid oder Butyl-lithium, gegebenenfalls unter Verwendung eines Verdünnungsmittels, wie z.B. Toluol, Tetrahydrofuran oder Acetonitril, bei Temperaturen zwischen -78 und 110 ° C umsetzt.
b) Man erhält die Verbindungen der Formel (I) weiterhin, wenn man die an sich bekannten oder nach bekannten Verfahren herstellbaren α,ω-Aminoalkohole bzw. α,ω-Aminoether der Formel (X)

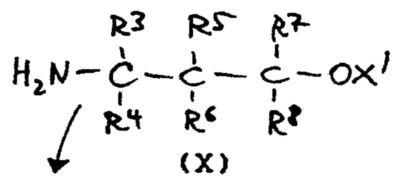

(X)

worin $R^3$ bis $R^8$, die unter Formel (I) angegebene Bedeutung haben und wobei X' für Wasserstoff oder $R^{13}$ steht,
zunächst mit an sich bekannten Chlorkohlensäureestern der Formel (III) gegebenenfalls in Gegenwart eines Säureakzeptors, wie z.B. Triethylamin oder Kaliumcarbonat und gegebenenfalls unter Verwendung eines Verdünnungsmittels, wie z.B. Toluol, $CH_2Cl_2$, Tetrahydrofuran oder Acetonitril, bei Temperaturen zwischen -40 ° C und 110 ° C, umsetzt.

In einem zweiten Reaktionsschritt wird dann für die Herstellung von Verbindungen der Formel (I), in denen X nicht für $R^{13}$ oder Wasserstoff steht, gegebenenfalls nach Isolierung des Zwischenproduktes mit freier OH-Gruppe,
die weitere Acylierung mit an sich bekannten Carbonsäurechloriden der Formel (IV) zur Herstellung von Verbindungen der Formel (I) mit X = $COR^{11}$, wobei in der Formel (IV), $R^{11}$ die obengenannte Bedeutung hat,
gegebenenfalls in Gegenwart eines Säureakzeptors wie z.B. Triethylamin oder Kaliumcarbonat, gegebenenfalls unter Verwendung eines Verdünnungsmittels, wie z.B. Toluol, Tetrahydrofuran oder Acetonitril, bei Temperaturen zwischen -78 und 110 ° C durchgeführt.

In einem dritten Reaktionsschritt wird dann, gegebenenfalls nach Isolierung des Zwischenproduktes mit freier NH-Gruppe, die weitere N-Alkylierung mit Alkylhalogeniden der Formel (XI)

$R^2$-Y' (XI),

zur Herstellung von Verbindungen der Formel (I), wobei Y' für Chlor, Brom oder Iod, vorzugsweise für Brom oder Iod steht und $R^2$ die obengenannte Bedeutung hat,
gegebenenfalls in Gegenwart einer Base wie z.B. Natriumhydrid oder Butyl-lithium, gegebenenfalls unter Verwendung eines Verdünnungsmittels, wie z.B. Toluol oder Tetrahydrofuran, bei Temperaturen zwischen -78 und 110° C, durchgeführt.

Die Aufarbeitung erfolgt nach üblichen Methoden, beispielsweise durch Extraktion der Produkte mit Methylenchorid oder Toluol aus der mit Wasser verdünnten Reaktionsmischung, Waschen der organischen Phase mit Wasser, Trocknen und Destillieren oder sogenanntes "Andestillieren", d.h. durch längeres Erhitzen unter vermindertem Druck auf mäßig erhöhte Temperaturen, um von den letzten flüchtigen Bestandteilen zu befreien.

Eine weitere Reinigung kann durch Chromatographie an Kieselgel mit z.B. Hexan:Aceton = 7:3 als Laufmittel erfolgen.

Zur Charakterisierung der Verbindungen dienen Brechungsindex, Schmelzpunkt, Rf-Wert oder Siedepunkt.

Die vorliegende Erfindung betrifft auch neue substituierte Aminoalkohol-Derivate der Formel (VII)

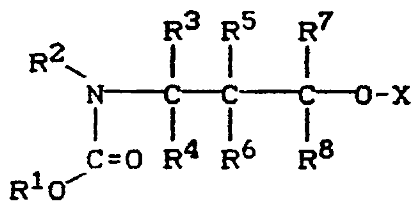
(VII)

in welcher

X für Wasserstoff, $COR^{11}$ oder $R^{13}$ steht,

$R^1$ für $C_1$-$C_7$-Alkyl, $C_3$-$C_7$-Alkenyl oder $C_2$-$C_7$-Alkinyl steht,

$R^2$, $R^{11}$ und $R^{13}$ gleich oder verschieden sind und für $C_1$-$C_6$-Alkyl oder $C_2$-$C_7$-Alkenyl stehen,

$R^3$ bis $R^8$ gleich oder verschieden sind und für Wasserstoff oder für $C_1$-$C_6$-Alkyl stehen, wobei $R^2$ und $R^3$ oder $R^3$ und $R^7$ oder $R^3$ und $R^5$ oder $R^5$ und $R^7$ auch gemeinsam mit den Atomen, an welche sie gebunden sind, einem 5- oder 6-gliedrigen monocyclischen Ring bilden können,

mit Ausnahme folgender Substituentenkombinationen a) bis f):

a) X = Wasserstoff, $R^2$ = Methyl und $R^1$ = tert. Butyl

b) X = Wasserstoff, $R^1$ = Ethyl, $R^5$ = Ethyl, $R^6$ = Ethyl,

c) X, $R^3$, $R^4$, $R^7$, $R^8$ = Wasserstoff, $R^1$, $R^5$, $R^6$ = Ethyl, $R^2$ = Methyl

d) X, $R^3$, $R^4$, $R^8$ = Wasserstoff, $R^1$, $R^2$, $R^5$, $R^6$ = Ethyl, $R^7$ = Methyl

e) X, $R^3$, $R^4$, $R^7$, $R^8$ = Wasserstoff, $R^1$, $R^2$, $R^5$, $R^6$ = Ethyl,

f) X, $R^3$, $R^4$, $R^7$, $R^8$ = Wasserstoff, $R^1$, $R^5$, $R^6$, = Ethyl $R^2$ = n-Propyl.

Man erhält die Verbindungen der Formel (VII),

a) wenn man die an sich bekannten oder nach bekannten Verfahren herstellbaren $\alpha,\omega$-Aminoalkohole (vergl. z. B. Cesare Ferri, Reaktionen der org. Synthese, Georg Thieme Verlag Stuttgart, 1978, S. 211 ff bzw. 496-497) der Formel

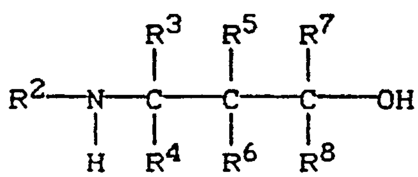
(VIII)

worin

$R^2$ bis $R^8$ die unter Formel (VII) angegebene Bedeutung haben,

zunächst mit an sich bekannten Kohlensäurederivaten der Formel (IX)

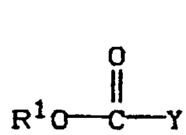
(IX)

wobei $R^1$ die unter Formel VII angegebene Bedeutung hat und Y für Halogen oder eine bei Amidierungsreaktionen übliche Abgangsgruppe steht, vorzugsweise einen aktivierenden Esterrest oder eine Gruppe

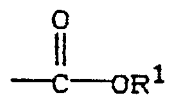

gegebenenfalls in Gegenwart eines Verdünnungsmittels und gegebenenfalls unter Zugabe einer Base umsetzt.

Für die Herstellung von Verbindungen der allgemeinen Formel (VII), in denen X verschieden von Wasserstoff ist, erfolgt dann in einem zweiten Reaktionsschritt, gegebenenfalls nach Isolierung des Zwischenproduktes mit freier OH-Gruppe, die weitere Acylierung/Alkylierung mit an sich bekannten Carbonsäurechloriden der Formel (IV)

$R^{11}COCl$ (IV)

zur Herstellung von Verbindungen der Formel (VII) mit X = $COR^{11}$
oder Alkylhalogeniden der Formel (VI)

$R^{13}$-Y (VI),

zur Herstellung von Verbindungen der Formel (I) mit X = $R^{13}$;
wobei in den Formeln (IV), (VI) Y für Chlor, Brom oder Iod, vorzugsweise für Brom oder Iod steht und $R^{11}$ und $R^{13}$ die obengenannte Bedeutung haben,
gegebenenfalls in Gegenwart eines Säureakzeptors wie z.B. Triethylamin oder Kaliumcarbonat oder einer Base wie Natriumhydrid oder Butyl-lithium, gegebenenfalls unter Verwendung eines Verdünnungsmittels, wie z. B. Toluol, Tetrahydrofuran oder Acetonitril, bei Temperaturen zwischen -78 und 110 °C umsetzt.

b) Man erhält die Verbindungen der Formel (VII) weiterhin, wenn man die an sich bekannten oder nach bekannten Verfahren herstellbaren α,ω-Aminoalkohole bzw. α,ω-Aminoether der Formel (XII)

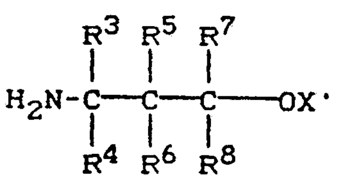

(XII)

worin $R^3$ bis $R^8$ die unter der Formel (VII) angegebene Bedeutung besitzen und wobei X' für Wasserstoff oder $R^{13}$ steht, wobei $R^{13}$ für gegebenenfalls substituiertes Alkyl oder Alkenyl steht,
zunächst mit an sich bekannten Chlorkohlensäureestern der Formel (III) gegebenenfalls in Gegenwart eines Säureakzeptors, wie z.B. Triethylamin oder Kaliumcarbonat und gegebenenfalls unter Verwendung eines Verdünnungsmittels wie z.B. Toluol, $CH_2Cl_2$, Tetrahydrofuran oder Acetonitril, vorzugsweise bei Temperaturen zwischen -40 °C und 110 °C, umsetzt.

In einem zweiten Reaktionsschritt wird dann für die Herstellung von Verbindungen der Formel (VII), in denen X nicht für $R^{13}$ oder Wasserstoff steht, gegebenenfalls nach Isolierung des Zwischenproduktes mit freier OH-Gruppe, die weitere Acylierung mit an sich bekannten Carbonsäurechloriden der Formel (IV) zur Herstellung von Verbindungen der Formel (VII) mit X = $COR^{11}$, wobei in den Formeln (IV), $R^{11}$ und $R^{12}$ die obengenannte Bedeutung haben,
gegebenenfalls in Gegenwart eines Säureakzeptors wie z.B. Triethylamin oder Kaliumcarbonat, gegebenenfalls unter Verwendung eines Verdünnungsmittels, wie z.B. Toluol, Tetrahydrofuran oder Acetonitril, bei Temperaturen zwischen -78 und 110 °C durchgeführt.

In einer dritten Reaktion wird dann, gegebenenfalls nach Isolierung des Zwischenproduktes mit freier NH-Gruppe, die weitere N-Alkylierung mit Alkylhalogeniden der Formel (XI)

$R^2$-Y' (XI),

zur Herstellung von Verbindungen der Formel (VII), wobei Y' für Chlor, Brom oder Iod, vorzugsweise für

Brom oder Iod steht und $R^2$ die obengenannte Bedeutung hat,
gegebenenfalls in Gegenwart einer Base wie z.B. Natriumhydrid oder Butyl-lithium, gegebenenfalls unter Verwendung eines Verdünnungsmittels, wie z.B. Toluol oder Tetrahydrofuran, bei Temperaturen zwischen -78 und 110 °C, durchgeführt.

Die Aufarbeitung erfolgt nach üblichen Methoden, beispielsweise durch Extraktion der Produkte mit Methylenchlorid oder Toluol aus der mit Wasser verdünnten Reaktionsmischung, Waschen der organischen Phase mit Wasser, Trocknen und Destillieren oder sogenanntes "Andestillieren", d. h. durch längeres Erhitzen unter vermindertem Druck auf mäßig erhöhte Temperaturen, um von den letzten flüchtigen Bestandteilen zu befreien.

Eine weitere Reinigung kann durch Chromatographie an Kieselgel mit z. B. Hexan : Aceton = 7:3 als Laufmittel erfolgen.

Die neuen substituierten $\alpha,\omega$-Aminoalkohol-Derivate der allgemeinen Formel (VII) zeichnen sich durch eine starke insekten- und milbenabweisende Wirkung aus. Sie können auch in synergistischen Mischungen mit anderen Repellentien verwendet werden.

Die in der Formel (VII) angegebenen Reste haben vorzugsweise die folgende Bedeutung:
Für die Alkylgruppe in den Resten $R^1$ bis $R^{13}$ seien beispielhaft genannt: Methyl, Ethyl, n- und i-Propyl, n-, i- und t-Butyl, n-Pentyl und n-Hexyl.

Für Alkenyl seien beispielhaft genannt: Propenyl-(2), Butenyl-(2) und Butenyl-(3).

Besonders bevorzugt sind Verbindungen der allgemeinen Formel (VII)
in welcher

| | |
|---|---|
| X | für Wassserstoff oder $R^{13}$ steht, wobei $R^{13}$ für $C_1$-$C_6$-Alkyl steht, |
| $R^1$ | für $C_1$-$C_7$-Alkyl oder $C_3$-$C_7$-Alkenyl steht, |
| $R^4$ bis $R^8$ | gleich oder verschieden sind und für Wasserstoff oder $C_1$-$C_6$-Alkyl stehen, |
| $R^2$ und $R^3$ | gemeinsam mit den Atomen, an welche sie gebunden sind, einen 5- oder 6-gliedrigen monocyclischen Ring bilden. |

Weiterhin werden Verbindungen der Formel VII bevorzugt, in denen $R^1$ für $C_1$-$C_7$-Alkyl, $C_3$-$C_7$-Alkenyl oder $C_2$ - $C_7$-Alkinyl steht, X für Wasserstoff, $COR^{11}$ oder $R^{13}$ steht, $R^2$ und $R^{11}$ gleich oder verschieden sind und für $C_1$-$C_6$-Alkyl stehen, $R^3$ bis $R^8$ gleich oder verschieden sind und für Wasserstoff oder $C_1$-$C_6$-Alkyl stehen, $R^{13}$ für $C_1$-$C_6$-Alkyl steht,
mit Ausnahme folgender Substituentenkombinationen a) bis f):

a) X = Wasserstoff, $R^2$ = Methyl und $R^1$ = tert. Butyl,
b) X = Wasserstoff, $R^1$, $R^5$ und $R^6$ = Ethyl,
c) X, $R^3$, $R^4$, $R^7$, $R^8$ = Wasserstoff, $R^1$, $R^5$, $R^6$ = Ethyl, $R^2$ = Methyl,
d) X, $R^3$, $R^4$, $R^8$ = Wasserstoff, $R^1$, $R^2$, $R^5$, $R^6$ = Ethyl, $R^7$ = Methyl
e) X, $R^3$, $R^4$, $R^7$, $R^8$ = Wasserstoff, $R^1$, $R^2$, $R^5$, $R^6$ = Ethyl,
f) X, $R^3$, $R^4$, $R^7$, $R^8$ = Wasserstoff, $R^1$, $R^5$, $R^6$ = Ethyl $R^2$ = n-Propyl.

Die Verbindungen der allgemeinen Formel (VII) enthalten ein oder mehrere Asymmetriezentren und können somit in Form von Diastereomeren oder Diastereomerengemischen vorliegen.

Verwendet man beispielsweise 2-(2-Hydroxyethyl)-piperidin und Chlorameisensäure-butylester als Ausgangsstoffe, so kann die Reaktion dieser Verbindungen durch folgendes Formelschema skizziert werden:

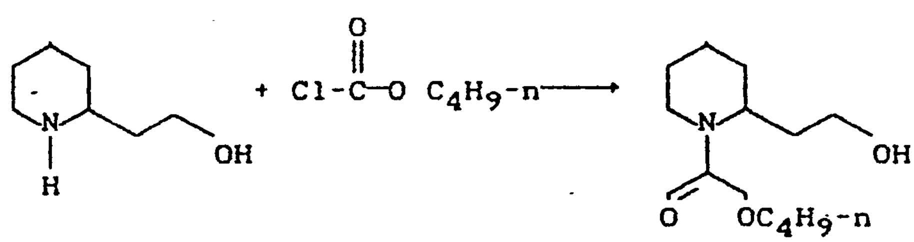

Die bei der Herstellung der neuen Verbindungen der Formel (VII) als Ausgangsprodukte eingesetzten Verbindungen der allgemeinen Formeln (VIII), (IX), (IV), (VI) sind allgemein bekannte Verbindungen der organischen Chemie oder können nach bekannten Verfahren und Methoden hergestellt werden (vgl. auch Herstellungsbeispiele).

Als Verdünnungsmittel für das erfindungsgemäße Verfahren kommen praktisch alle organischen Verdünnungsmittel in Frage, welche sich unter den Verfahrensbedingungen inert verhalten. Hierzu gehören insbesondere aliphatische und aromatische, gegebenenfalls halogenierte Kohlenwasserstoffe, wie Pentan,

Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glykoldimethylether und Diglykoldimethylether, Tetrahydrofuran und Dioxan, Ester, wie Essigsäuremethylester und -ethylester, Nitrile, wie z. B. Acetonitril und Propionitril, Amide, wie z. B. Dimethylformamid, Dimethylacetamid und N-Methylpyrrolidon, sowie Dimethylsulfoxid, Tetramethylensulfon und Hexamethylphosphorsäuretriamid.

Die Reaktionstemperaturen können in einem größeren Bereich variiert werden. Bevorzugt arbeitet man bei Temperaturen zwischen -50° C und 120° C, vorzugsweise zwischen -20° C und 110° C. Die Umsetzungen werden im allgemeinen bei Normaldruck durchgeführt.

Die Reaktionen werden vorzugsweise in Gegenwart basischer Hilfsstoffe durchgeführt. Die jeweils günstigste Basenmenge kann leicht experimentell ermittelt werden. Als Basen kommen vorzugsweise solche Basen in Frage, welche üblicherweise auch als Säurebindemittel verwendet werden. Beispielsweise seien aufgeführt:

Alkalicarbonate und -alkoholate, wie Natrium- und Kaliumcarbonat, Natrium- und Kaliummethylat, -ethylat bzw. t-Butylat, ferner aliphatische, aromatische oder heterocyclische Amine, beispielsweise Triethylamin, Trimethylamin, Dimethylanilin, Dimethylbenzylamin und Pyridin sowie Metallhydride wie Natriumhydrid oder metallorganische Verbindungen wie z. B. n-Butyllithium.

Zur Durchführung des erfindungsgemäßen Verfahrens a) setzt man zunächst vorzugsweise auf ein Mol $\alpha,\omega$-Aminoalkohol der Formel (VIII) 1 bis 2 Mol, insbesondere 1 bis 1,2 Mol der Verbindung (IX) ein.

In gleicher Weise werden bei der Herstellung von Verbindungen der Formel (VII), in denen X verschieden von Wasserstoff ist, gegebenenfalls nach Isolierung des Zwischenproduktes mit freier OH-Gruppe, 1 bis 2 Mol, vorzugsweise 1 bis 1,2 Mol der Verbindungen (IV) oder (VI) eingesetzt. Falls eine Base eingesetzt wird, wird diese mit 1 bis 4 Mol, vorzugsweise 1 bis 1,5 Mol je Mol des Zwischenproduktes verwendet.

Zur Durchführung des erfindungsgemäßen Verfahrens b) setzt man zunächst vorzugsweise auf ein Mol Amin der Formel (XII) 1 bis 2 Mol, insbesondere 1 bis 1,2 Mol der Verbindung (III) ein.

In gleicher Weise werden bei der Herstellung von Verbindungen der Formel (VII), in denen X nicht für Wasserstoff oder $R^{13}$ steht, gegebenenfalls nach Isolierung des Zwischenproduktes mit freier OH-Gruppe, 1 bis 2 Mol, vorzugsweise 1 bis 1,2 Mol der Verbindung (IV) eingesetzt. Falls eine Base eingesetzt wird, wird diese mit 1 bis 4 Mol, vorzugsweise 1 bis 1,5 Mol je Mol des Zwischenproduktes verwendet.

Zur nachfolgenden N-Alkylierung werden gegebenenfalls nach vorheriger Isolierung auf 1 Mol des NH-Zwischenproduktes, 1 bis 10 Mol, vorzugsweise 1 bis 1,5 Mol, der Verbindung $R^2$-$Y'$ eingesetzt. Falls eine Base erforderlich ist, wird diese mit 1 bis 4 Mol, vorzugsweise mit 1 bis 1,5 Mol je Mol des NH-Zwischenproduktes verwendet.

Die Aufarbeitung der erfindungsgemäßen Verbindungen der Formel (VII) geschieht in analoger Weise, wie weiter vorne, bei der Herstellung der Verbindungen der Formel (I) beschrieben.

Die Wirkung der Repellentien der allgemeinen Formel (I bzw. VII) hält lange an.

Sie können daher mit gutem Erfolg zur Abwehr von schädlichen oder lästigen, saugenden und beißenden Insekten und Milben verwendet werden.

Zu den saugenden Insekten gehören im wesentlichen die Stechmücken (z.B. Aedes-, Culex- und Anopheles-Arten), Schmetterlingsmücken (Phlebotomen), Gnitzen (Culicoides-Arten), Kriebelmücken (Simulium-Arten), Stechfliegen (z.B. Stomoxys calcitrans), Tsetse-Fliegen (Glossina-Arten), Bremsen (Tabanus-, Haematopota- und Chrysops-Arten), Stubenfliegen (z.B. Musca domestica und Fannia canicularis), Fleischfliegen (z.B. Sarcophaga carnaria), Myiasis erzeugende Fliegen (z.B. Lucilia cuprina, Chrysomyia chloropyga, Hypoderma bovis, Hypoderma lineatum, Dermatobia hominis, Oestrus ovis, Gasterophilus intestinalis, Cochliomyia hominovorax), Wanzen (z.B. Cimex lectularius, Rhodnius prolixus, Triatoma infestans), Läuse (z.B. Pediculus humanus, Haematopinus suis, Damalina ovis), Lausfliegen (z.B. Melaphagus orinus), Flöhe (z.B. Pulex irritans, Cthenocephalides canis, Xenopsylla cheopis) und Sandflöhe (z.B. Dermatophilus penetrans).

Zu den beißenden Insekten gehören im wesentlichen Schaben (z.B. Blattella germanica, Periplaneta americana, Blatta orientalis, Supella supellectilium), Käfer (z.B. Sitophilus granarius, Tenebrio molitor, Dermestes lardarius, Stegobium paniceum, Anobium puntactum, Hylotrupes bajulus), Termiten (z.B. Reticulitermes lucifugus) und Ameisen (z.B. Lasius niger).

Zu den Milben gehören Zecken (z.B. Ornithodorus moubata, Ixodes ricinus, Boophilus microplus, Amblyomma hebreum) und Milben in engerem Sinne (z.B. Sarcoptes scabiei, Dermanyssus gallinae).

Die erfindungsgemäßen Wirkstoffe, die unverdünnt oder vorzugsweise verdünnt eingesetzt werden können, lassen sich in die für Repellents üblichen Formulierungen überführen. Sie lassen sich in allen in der Kosmetik üblichen Darreichungsformen einsetzen, beispielsweise in Form von Lösungen, Emulsionen,

Gelen, Salben, Pasten, Cremes, Pulvern, Stiften, Sprays oder Aerosolen aus Sprühdosen.

Für die Anwendung im nichtkosmetischen Bereich lassen sich die Wirkstoffe z.B. in Granulate, Ölsprühmittel oder Slow-Release-Formulierungen einarbeiten.

Die Zubereitungen werden in bekannter Weise durch Vermischen oder Verdünnen der erfindungsgemäßen Wirkstoffe mit Lösungsmitteln, (z.B. Xylol, Chlorbenzole, Paraffine, Methanol, Ethanol, Isopropanol, Wasser), Trägerstoffe (z.B. Kaoline, Tonerden, Talkum, Kreide, hochdisperse Kieselsäure, Silikate), Emulgiermitteln (z.B. Polyoxyethylen-Fettsäure-Ester,Polyoxyethylen-Fettalkohol-Ether, Alkylsulfonate, Arylsulfonate) und Dispergiermitteln (z.B. Lignin-, Sulfitablaugen, Methylcellulose) hergestellt.

Die erfindungsgemäßen Wirkstoffe können in den Formulierungen miteinander gemischt oder auch in Mischungen mit anderen bekannten Wirkstoffen (z.B. Sonnenschutzmittel) eingesetzt werden. Die Zubereitungen enthalten im allgemeinen zwischen 0,1 und 95 Gew.-% Wirkstoff, vorzugsweise zwischen 0,5 und 90%.

Zum Schutz gegen blutsaugende Insekten oder Milben werden die erfindungsgemäßen Wirkstoffe entweder auf die menschliche oder tierische Haut aufgebracht oder Kleidungsstücke und andere Gegenstände damit behandelt.

Auch als Zusatz von Imprägniermitteln für beispielsweise Textilbahnen, Kleidungsstücke, Verpackungsmaterialien, sowie als Zusatz zu Polier-, Putz- und Fensterreinigungsmitteln sind die erfindungsgemäßen Wirkstoffe geeignet.

Die folgenden Beispiele für die Zubereitungen und die Verwendung der erfindungsgemäßen Wirkstoffe dienen der weiteren Erläuterung der Erfindung.

Formulierungs-Beispiel 1

Ein Repellentmittel in Form einer Lotion zur Anwendung auf der Haut wird hergestellt durch Vermischen von 30 Teilen eines der erfindungsgemäßen Wirkstoffe, 1,5 Teilen Parfüm und 68,5 Teilen Isopropanol. Isopropanol kann durch Ethanol ersetzt werden.

Formulierungs-Beispiel 2

Ein Repellentmittel in Form eines Aerosols zum Aufsprühen auf die Haut wird hergestellt, indem man 50 % Wirkstofflösung, bestehend aus 30 Teilen eines der erfindungsgemäßen Wirkstoffe, 1,5 Teilen Parfüm, 68,5 Teilen Isopropanol, mit 50 % Frigen 11/12 (= halogenierter Kohlenwasserstoff als Treibgas) als Sprühdosenpräparation formuliert.

Formulierungs-Beispiel 3

Eine andere Sprühdosenpräparation setzt sich aus 40 % Wirkstofflösung, bestehend aus 20 Teilen eines der erfindungsgemäßen Wirkstoffe, 1 Teil Parfüm, 79 Teilen Isopropanol und 60 % Propan/Butan (Verhältnis 15:85) zusammen.

Es wurden individuelle Formulierungen entsprechend den Formulierungs-Beispielen 1, 2 und 3 unter Einsatz folgender Wirkstoffe hergestellt: Verbindungen gemäß Herstellungsbeispielen Nr. 1, 2, 4, 5, 6.

Beispiel A

Repellenttest am Meerschweinchen
Testtier: Aedes aegypti (Imagines)
Zahl der Testtiere: ca. 5.000
Lösungsmittel: Ethanol (99,8 %)
3 Gewichtsteile Wirkstoff werden in 100 Volumenteilen Lösungsmittel aufgenommen.

Ein Meerschweinchen, dessen Rücken in einem Bereich von 50 $cm^2$ rasiert worden ist, wird in einem engen Käfig (Box) so fixierf, daß nur die rasierte Fläche den Mücken zugänglich ist. Nach Behandeln der Fläche mit 0,4 ml Wirkstofflösung wird das Meerschweinchen nach Verdunsten des Lösungsmittels samt Box in einen 60 x 60 x 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen.

Anschließend wird dieses herausgenommen und der Test nach einer Stunde wiederholt. Der Versuch wird maximal 9 Stunden lang durchgeführt oder so lange, bis die Wirkung abbricht.

Bei diesem Test zeigen z. B. die folgenden Verbindungen der Herstellungsbeispiele überlegene

Wirkung gegenüber dem Stand der Technik (Diethyltoluamid = Deet):

Tabelle A

Repellenttest am Meerschweinchen (Aedes aegypti)

| Präparat | | Anzahl der Einstiche nach: $0^h-6^h$ | $7^h-9^h$ |
|---|---|---|---|
| Erfindungsgemäß Hst.-Bsp. Nr. 1 | 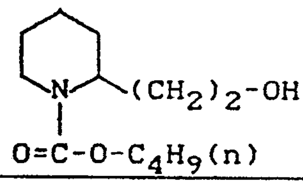 | 0 | 0,1 |
| Hst.-Bsp. Nr. 4 | 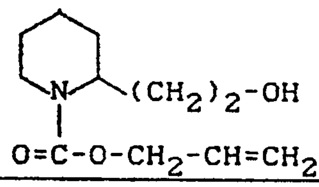 | 1,0 | 2,7 |
| Hst.-Bsp. Nr. 5 | 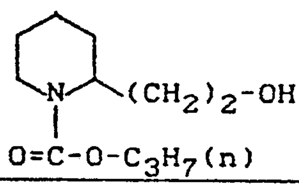 | 1,5 | 3,3 |
| Hst.-Bsp. Nr. 6 | 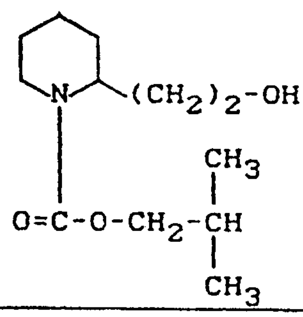 | 0,1 | 1,4 |

<u>Tabelle A</u> (Fortsetzung)

Repellenttest am Meerschweinchen (Aedes aegypti)

| Präparat | | Anzahl der Einstiche nach: $0^h$-$6^h$ | $7^h$-$9^h$ |
|---|---|---|---|
| Hst.-Bsp. Nr. 2 | 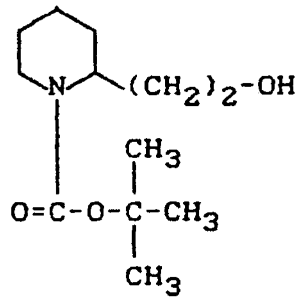 | 0 | 6,1 |
| Hst.-Bsp. Nr. 36 | 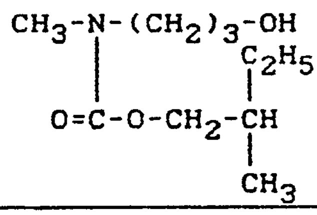 | 1,2 | 11,8 |
| Hst.-Bsp. Nr. 49 | 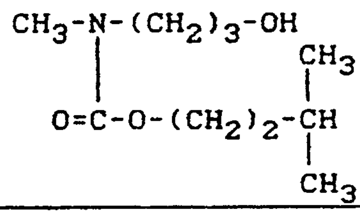 | 1,3 | 13,0 |
| Hst.-Bsp. Nr. 55 | 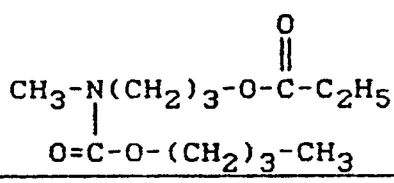 | 1,7 | 4,9 |
| Hst.-Bsp. Nr. 25 | 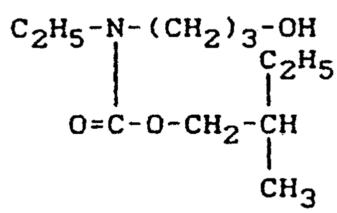 | 0,4 | 6,0 |

Tabelle A (Fortsetzung)

Repellenttest am Meerschweinchen (Aedes aegypti)

| Präparat | | Anzahl der Einstiche nach: $0^h$-$6^h$ | $7^h$-$9^h$ |
|---|---|---|---|
| Hst.-Bsp. Nr. 26 | 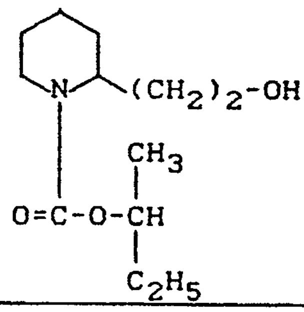 | 0 | 0,1 |
| Hst.-Bsp. Nr. 31 | 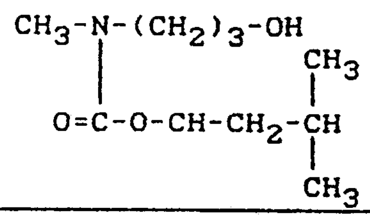 | 2,0 | 5,0 |
| Bekannt: Deet | 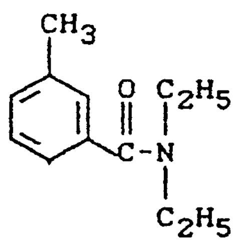 | 2,4 | 14,9 |

Anmerkung: "Hst.-Bsp." bedeutet "Herstellungsbeispiel"

Beispiel B

Repellenttest am Meerschweinchen
Testtier: Anopheles albimanus (Imagines)
Zahl der Testtiere: ca. 1.000
Lösungsmittel: Ethanol (99,8 %)
3 Gewichtsteile Wirkstoff werden in 100 Volumenteilen Lösungsmittel aufgenommen.

Ein Meerschweinchen, dessen Rücken in einem Bereich von 50 $cm^2$ rasiert worden ist, wird in einem engen Käfig (Box) so fixiert, daß nur die rasierte Fläche den Mücken zugänglich ist. Nach Behandeln der Fläche mit 0,4 ml Wirkstofflösung wird das Meerschweinchen nach Verdunsten des Lösungsmittels samt Box in einen 60 x 60 x 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen.

Anschließend wird dieses herausgenommen und der Test nach einer Stunde wiederholt. Der Versuch wird maximal 9 Stunden lang durchgeführt oder so lange, bis die Wirkung abbricht.

Bei diesem Test zeigen z. B. die folgenden Verbindungen der Herstellungsbeispiele überlegene Wirkung gegenüber dem Stand der Technik (Diethyltoluamid = Deet):

Tabelle B

Repellenttest am Meerschweinchen (Anopheles albimanus)

| Präparat | | Anzahl der Einstiche nach: | |
|---|---|---|---|
| | | $0^h-6^h$ | $7^h-9^h$ |
| Erfindungsgemäß Hst.-Bsp. Nr. 1 | 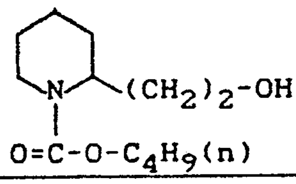 | 0,6 | 1,3 |
| Hst.-Bsp. Nr. 5 | 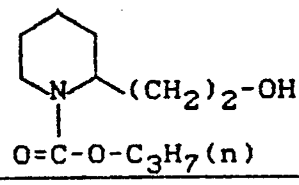 | 0,4 | 7,1 |
| Hst.-Bsp. Nr. 6 | 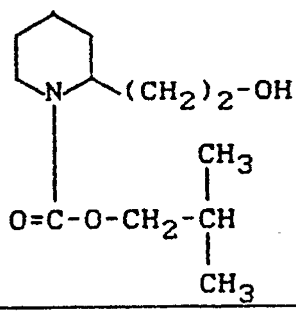 | 1,0 | 0,8 |

Tabelle B (Fortsetzung)

Repellenttest am Meerschweinchen (Anopheles albimanus)

| Präparat | | Anzahl der Einstiche nach: $0^h$-$6^h$ | $7^h$-$9^h$ |
|---|---|---|---|
| Hst.-Bsp. Nr. 2 | 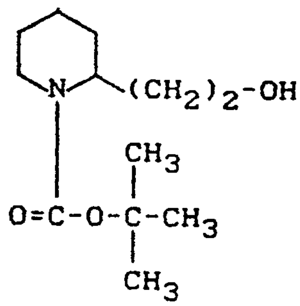 | 0 | 1,6 |
| Hst.-Bsp. Nr. 4 | 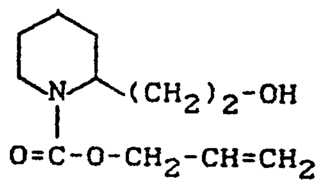 | 0,9 | 6,5 |
| Hst.-Bsp. Nr. 36 | 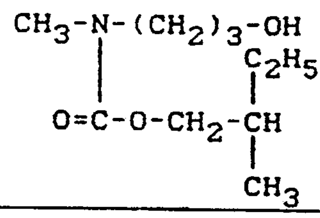 | 0 | 0,2 |
| Hst.-Bsp. Nr. 48 | 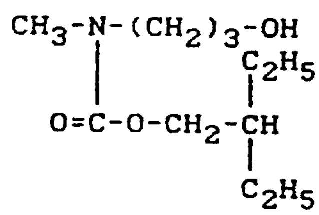 | 0 | 0 |

Tabelle B (Fortsetzung)

Repellenttest am Meerschweinchen (Anopheles albimanus)

| Präparat | | Anzahl der Einstiche nach: $0^h$-$6^h$ | $7^h$-$9^h$ |
|---|---|---|---|
| Hst.-Bsp. Nr. 49 | 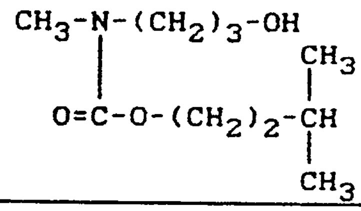 | 0,1 | 4,0 |
| Hst.-Bsp. Nr. 25 | 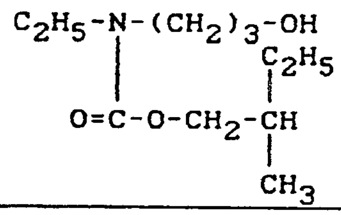 | 0,3 | 2,0 |
| Hst.-Bsp. Nr. 26 | 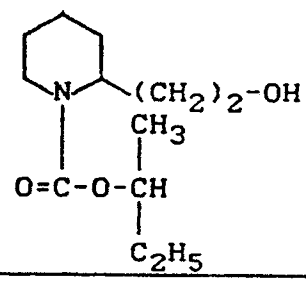 | 0,1 | 0,2 |
| Bekannt: Deet | 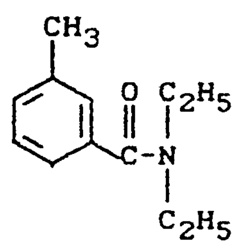 | 1,0 | 5,0 |

Herstellungsbeispiele

Beispiel 1

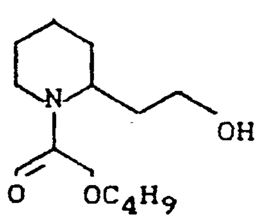

1-(Butoxycarbonyl)-2-(2-hydroxyethyl)-piperidin

6,5 g (0,05 Mol) 2-(2-Hydroxyethyl)-piperidin und 10 ml Triethylamin werden in 300 ml Tetrahydrofuran gelöst und bei -20 °C mit 7,5 g (0,55 Mol) Chlorameisensäurebutylester versetzt.

Man rührt 24 h bei 20 °C und extrahiert dann mit Methylenchlorid/Wasser. Nach Trocknen der organischen Phase mit Magnesiumsulfat wird das Lösungsmittel im Vakuum abdestilliert und anschließend der Rückstand im Kugelrohrofen destilliert.

Ausbeute: 5,6 g (= 49 % der Theorie)
Siedepunkt (Kugelrohr): 120-130 °C/0,25 mbar

Beispiel 2

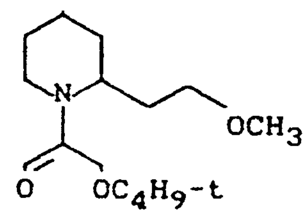

1-(t-Butoxycarbonyl)-2-(2-methoxyethyl)-piperidin

0,5 g 1-(t-Butoxycarbonyl)-2-(2-hydroxyethyl)-piperidin (0,002 Mol) werden in 50 ml Tetrahyrofuran und bei -78 °C mit 1,1 ml einer 23 %igen Butyllithiumlösung in Hexan versetzt.

Man erwärmt 1 Stunde auf 20 °C und 4 h auf 40 °C, kühlt auf 20 °C und gibt 5 ml Methyliodid (0,08 Mol) zur Reaktionsmischung. Man erwärmt noch 24 h auf 40 °C und gießt dann auf 300 ml Eiswasser. Anschließend wird mehrfach mit Methylenchlorid extrahiert. Nach Trocknen der organischen Phase mit Magnesiumsulfat und Abdestillieren des Lösungsmittels wird über ca. 200 g Kieselgel chromatographiert (Laufmittel : Hexan : Essigester = 7 : 3). Nach Abdestillieren des Lösungsmittels erhält man 0,3 g (= 62 % der Theorie) 1-(Butoxycarbonyl)-2-(2-methoxyethyl)-piperidin mit einem Brechungsindex $n_D^{20}$ = 1,4613.

Beispiel 3

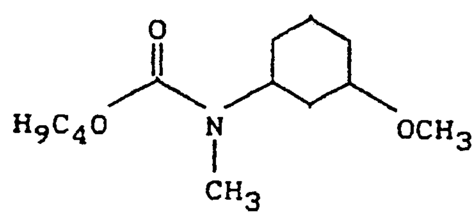

26 g (0,2 Mol) 1-Amino-3-methoxycyclohexan und 35 ml Triethylamin (0,25 Mol) werden in 500 ml Tetrahydrofuran gelöst und bei 20 °C mit 30 ml (0,24 Mol) Chlorameisensäurebutylester versetzt. Man rührt 1 d bei 20 °C, filtriert den Feststoff ab, destilliert das Lösungsmittel ab, nimmt in Methylenchlorid auf und filtriert über Kieselgel. Man erhält nach erneutem Abdestillieren des Lösungsmittels 36 g (78 % d. Theorie) 1-(N-Butoxycarbonyl-)amino-3-methoxycyclohexan.

Zur weiteren Umsetzung werden 11,5 g (0,05 Mol) dieser Verbindung in 150 ml Tetrahydrofuran gelöst und bei 20 °C mit 2,2 g (0,073 Mol) Natriumhydrid (80 %ig in Paraffin) versetzt. Man erwärmt 4 h auf Rückfluß und fügt dann bei 20 °C 10 ml (0,16 Mol) Methyliodid zur Reaktionsmischung. Man erwärmt 1 h auf Rückfluß, fügt zunächst 50 ml einer Ammoniumchloridlösung, dann Methylenchlorid/Wasser hinzu, trocknet die organische Phase mit Magnesiumsulfat und destilliert das Lösungsmittel ab. Anschließend wird mit Hexan: Aceton = 1:1 über Kieselgel filtriert. Man erhält 8,4 g (69 % d. Th.) 1-(N-Butoxycarbonyl-N-methyl)-3-methoxycyclohexan mit einem Brechungsindex $n_D^{20}$ = 1,4632.

Analog werden erhalten:

Allgemeine Formel: 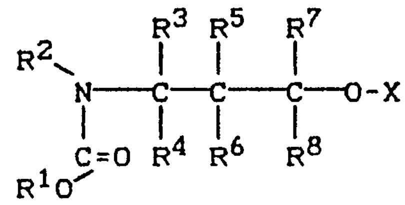 (VII)

| Herst. Bsp.Nr. | $R^1$ | $R^2$ | $R^3$ | $R^4$ | $R^5$ | $R^6$ | $R^7$ | $R^8$ | X | phys.Daten ($n_D^{20}$) |
|---|---|---|---|---|---|---|---|---|---|---|
| 4 | $CH_2=CH-CH_2-$ | $-CH_2-CH_2-CH_2-CH_2-$ | | H | H | H | H | H | H | 1,4893 |
| 5 | $n-C_3H_7$ | " | | " | " | " | " | " | " | 1,4761 |
| 6 | $(CH_3)_2CH-CH_2-$ | " | | " | " | " | " | " | " | 1,4710 |
| 7 | $n-C_4H_9-$ | $CH_3$ | H | " | " | " | " | " | H | 1,4506 |
| 8 | $(C_2H_5)CH(CH_3)CH_2$ | $-CH_2-CH_2-CH_2-CH_2-$ | | " | " | " | " | " | " | 1,4748 |
| 9 | $t-C_4H_9$ | $-CH_2-CH_2-CH_2-CH_2-$ | | " | " | " | " | " | " | 1,4705 |
| 10 | $n-C_4H_9$ | $n-C_4H_9$ | H | " | " | " | " | " | " | 1,4506 |
| 11 | $n-C_4H_9$ | $CH_3$ | H | " | " | " | " | " | $COCH_3$ | 1,4423 |

Allgemeine Formel: 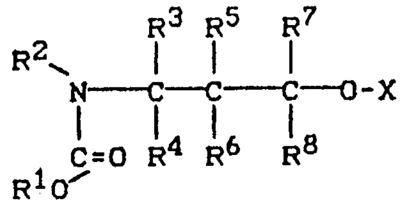 (VII)

| Herst. Bsp.Nr. | $R^1$ | $R^2$ | $R^3$ | $R^4$ | $R^5$ | $R^6$ | $R^7$ | $R^8$ | X | phys.Daten ($n_D^{20}$) |
|---|---|---|---|---|---|---|---|---|---|---|
| 12 | $n-C_4H_9$ | $t-C_4H_9$ | H | H | H | H | H | H | H | |
| 13 | " | " | " | " | " | " | " | " | $COCH_3$ | |
| 14 | " | $n-C_4H_9$ | " | " | " | " | " | " | $COCH_3$ | 1,4436 |
| 15 | $C_2H_5$ | $t-C_4H_9$ | " | " | " | " | " | " | $COCH_3$ | 1,4551 |
| 16 | $(CH_3)_2CHCH_2$ | $t-C_4H_9$ | " | " | " | " | " | " | H | |
| 17 | $(CH_3)_3CCH_2$ | $-CH_2-CH_2-CH_2-CH_2-$ | | " | " | " | " | " | " | Fp. 51° C |
| 18 | $(CH_3)_2CHCH_2CH_2$ | " | | " | " | " | " | " | " | 1,4730 |
| 19 | $C_2H_5$ | $t-C_4H_9$ | H | " | " | " | " | " | " | |
| 20 | $(C_2H_5)_2CHCH_2$ | $-CH_2-CH_2-CH_2-CH_2-$ | | " | " | " | " | " | " | 1,4750 |
| 21 | $n-C_4H_9$ | $CH_3$ | H | " | " | " | " | " | $CH_3$ | |

Allgemeine Formel: 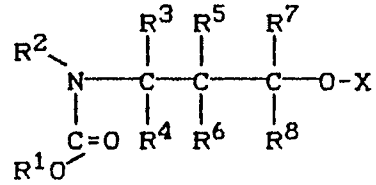 (VII)

| Herst. Bsp.Nr. | $R^1$ | $R^2$ | $R^3$ | $R^4$ | $R^5$ | $R^6$ | $R^7$ | $R^8$ | X | phys.Daten ($n_D^{20}$) |
|---|---|---|---|---|---|---|---|---|---|---|
| 22 | $t-C_4H_9$ | $CH_3$ | H | H | H | H | H | H | H | 1,4415 ($Kp._{0,08}$ 120° C Kugelrohr) |
| 23 | " | " | " | " | " | " | " | " | $COCH_3$ | 1,4261 ($Kp._{0,05}$ 110° C Kugelrohr) |
| 24. | $n-C_4H_9$ | 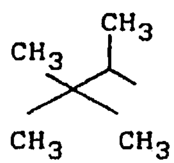 | " | " | " | " | " | " | $COCH_3$ | 1,4534 |
| 25 | $(C_2H_5)CH(CH_3)CH_2$ | $C_2H_5$ | " | " | " | " | " | " | H | 1,4490 |
| 26 | $(C_2H_5)(CH_3)CH$ | $-(CH_2)_4-$ | | " | " | " | " | " | " | 1,4717 |
| 27 | $(n-C_3H_7)(CH_3)CH$ | $-(CH_2)_4-$ | | " | " | " | " | " | " | 1,4097 |
| 28 | $CH_3OCH_2CH_2$ | $CH_3$ | " | " | " | " | " | " | " | 1,4544 |
| 29 | $(n-C_3H_7)(CH_3)CH$ | $CH_3$ | " | " | " | " | " | " | " | 1,4458 |

Allgemeine Formel: 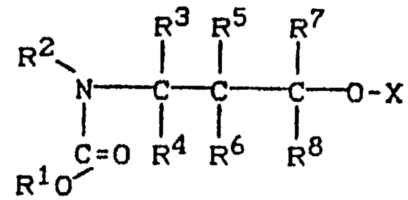 (VII)

| Herst. Bsp.Nr. | $R^1$ | $R^2$ | $R^3$ | $R^4$ | $R^5$ | $R^6$ | $R^7$ | $R^8$ | X | phys.Daten ($n_D^{20}$) |
|---|---|---|---|---|---|---|---|---|---|---|
| 30 | $(C_2H_5)(CH_3)CH$ | $CH_3$ | H | H | H | H | H | H | H | 1,4452 |
| 31 | $(i\text{-}C_3H_7)CH_2(CH_3)CH$ | $CH_3$ | " | " | " | " | " | " | " | 1,4457 |
| 32 | $(i\text{-}C_3H_7)CH_2(CH_3)CH$ | $-(CH_2)_4-$ | | " | " | " | " | " | " | 1,7064 |
| 33 | $(n\text{-}C_3H_7)(CH_3)CH$ | $CH_3$ | " | " | " | " | " | " | $COCH_3$ | 1,4450 |
| 34 | $(C_2H_5)(CH_3)CH$ | $CH_3$ | " | " | " | " | " | " | $COCH_3$ | 1,4443 |
| 35 | $(i\text{-}C_3H_7)CH_2(CH_3)CH$ | $CH_3$ | " | " | " | " | " | " | $COCH_3$ | 1,4455 |
| 36 | $(C_2H_5)CH(CH_3)CH_2$ | $CH_3$ | " | " | " | " | " | " | H | 1,4715 |
| 37 | $(C_2H_5)CH(CH_3)CH_2$ | $C_2H_5$ | " | " | " | " | " | " | " | 1,4490 |
| 38 | $(C_2H_5)CH(CH_3)CH_2$ | $n\text{-}C_3H_7$ | " | " | " | " | " | " | " | |
| 39 | $(C_2H_5)CH(CH_3)$ | $-CH_2CH_2CH(CH_3)CH_2$ | | " | " | " | " | " | " | |

Allgemeine Formel: 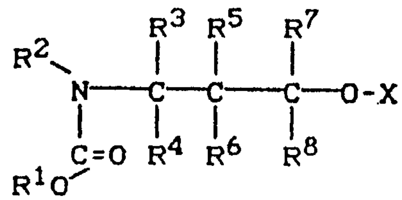 (VII)

| Herst. Bsp.Nr. | $R^1$ | $R^2$ | $R^3$ | $R^4$ | $R^5$ | $R^6$ | $R^7$ | $R^8$ | X | phys.Daten ($n_D^{20}$) |
|---|---|---|---|---|---|---|---|---|---|---|
| 40 | $n\text{-}C_4H_9$ | $-CH_2CH_2CH(CH_3)CH_2-$ | | H | H | H | H | H | H | |
| 41 | $(C_2H_5)CH(CH_3)$ | $-CH_2CH(C_2H_5)CH_2CH_2-$ | | " | " | " | " | " | " | |
| 42 | $n\text{-}C_4H_9$ | $-CH_2CH(C_2H_5)CH_2CH_2-$ | | " | " | " | " | " | " | |
| 43 | $(C_2H_5)CH(CH_3)CH_2$ | $CH_3$ | $CH_3$ | " | " | " | " | " | " | |
| 44 | $(C_2H_5)CH(CH_3)CH_2$ | $C_2H_5$ | $CH_3$ | " | " | " | " | " | " | |
| 45 | $CH_3$ | $n\text{-}C_4H_9$ | H | " | " | " | " | " | " | |
| 46 | $CH_3$ | $CH_3\text{-}CH{=}CH\text{-}CH_2$ | " | " | " | " | " | " | " | |
| 47 | $CH_3$ | $CH_2{=}CH\text{-}CH_2$ | " | " | " | " | " | " | " | |
| 48 | $(C_2H_5)_2CHCH_2$ | $CH_3$ | " | " | " | " | " | " | " | 1,4533 |
| 49 | $(CH_3)_2CHCH_2CH_2$ | $CH_3$ | " | " | " | " | " | " | " | 1,4508 |

Allgemeine Formel: 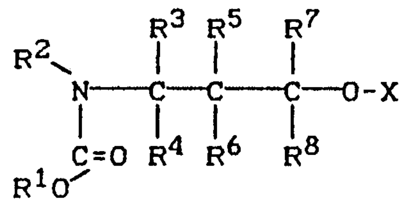 (VII)

| Herst. Bsp.Nr. | $R^1$ | $R^2$ | $R^3$ | $R^4$ | $R^5$ | $R^6$ | $R^7$ | $R^8$ | X | phys.Daten ($n_D^{20}$) |
|---|---|---|---|---|---|---|---|---|---|---|
| 50 | $(CH_3)_3CCH_2$ | $CH_3$ | H | H | H | H | H | H | H | 1,4471 |
| 51 | $(C_2H_5)_2CHCH_2$ | $CH_3$ | " | " | " | " | " | " | $COCH_3$ | 1,4469 |
| 52 | $(C_2H_5)(CH_3)CHCH_2$ | $CH_3$ | " | " | " | " | " | " | $COCH_3$ | 1,4450 |
| 53 | $(CH_3)_2CHCH_2CH_2$ | $CH_3$ | " | " | " | " | " | " | $COCH_3$ | 1,4426 |
| 54 | $(CH_3)_3CCH_2$ | $CH_3$ | " | " | " | " | " | " | $COCH_3$ | 1,4406 |
| 55 | $CH_3-(CH_2)_2-CH_2$ | $CH_3$ | " | " | " | " | " | " | $COC_2H_5$ | |
| 56 | $CH_2=CH-CH_2CH_2-$ | $-(CH_2)_4-$ | | " | " | " | " | " | H | 1,4920 |
| 57 | $CH_3-CH=CH-CH_2-$ | $-(CH_2)_4-$ | | " | " | " | " | " | H | 1,9420 |
| 58 | $CH_2=CH-CH_2CH_2-$ | $CH_3$ | H | " | " | " | " | " | H | 1.4625 |
| 59 | $CH_3-CH=CH-CH_2-$ | $CH_3$ | " | " | " | " | " | " | H | 1.4690 |

Allgemeine Formel: 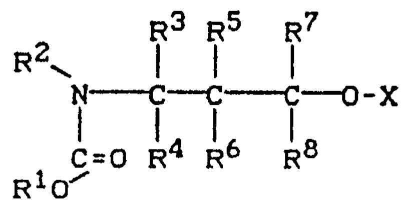 (VII)

| Herst. Bsp.Nr. | $R^1$ | $R^2$ | $R^3$ | $R^4$ | $R^5$ | $R^6$ | $R^7$ | $R^8$ | X | phys.Daten ($n_D^{20}$) |
|---|---|---|---|---|---|---|---|---|---|---|
| 60 | $CH_3$-CH=CH-$CH_2$- | $CH_3$ | H | H | H | H | H | H | $COCH_3$ | 1,4576 |
| 61 | $CH_2$=CH-$CH_2$-$CH_2$- | $CH_3$ | " | " | " | " | " | " | $COCH_3$ | 1,4522 |
| 62 | $CH_3$-C≡C-$CH_2$- | -$(CH_2)_4$- | | " | " | " | " | " | H | 1,4986 |
| 63 | 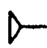$CH_2$ | -$(CH_2)_4$- | | " | " | " | " | " | H | 1,4835 |

Herstellung von Verbindungen der allgemeinen Formel (VIII)

N-Butylaminopropanol-1,3

500 ml Butylamin und 1 g Kaliumiodid werden mit 48 g (0,5 Mol) Chlorpropanol-1,3 versetzt und 2 Tage auf Rückfluß erhitzt. Anschließend wird mit Methylenchlorid/Wasser extrahiert, die organische Phase mit Magnesiumsulfat getrocknet, einrotiert und destilliert. Man erhält 52 g (= 79 % der Theorie) N-Butylamino-propanol-1,3 mit einem Siedepunkt: Kp. 125 °C / 30 mbar.

**Patentansprüche**

1. Insekten- und milbenabwehrende Mittel, gekennzeichnet durch einen Gehalt an mindestens einem substituierten $\alpha,\omega$-Aminoalkohol-Derivat der Formel (I)

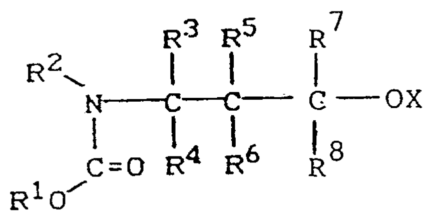

(I)

in welcher

| | |
|---|---|
| X | für Wasserstoff, $COR^{11}$ oder $R^{13}$ steht, |
| $R^1$ | für $C_1$-$C_7$-Alkyl, $C_3$-$C_7$-Alkenyl oder $C_2$-$C_7$-Alkinyl steht, |
| $R^2$,$R^{11}$,$R^{13}$ | gleich oder verschieden sind und für $C_1$-$C_6$-Alkyl oder $C_2$-$C_7$-Alkenyl stehen, |
| $R^3$-$R^8$ | gleich oder verschieden sind und für Wasserstoff oder $C_1$-$C_6$-Alkyl stehen, wobei $R^2$ und $R^3$ oder $R^3$ und $R^7$ oder $R^3$ und $R^5$ oder $R^5$ und $R^7$ gemeinsam mit den Atomen, an welche sie gebunden sind, auch einen 5- oder 6-gliedrigen monocyclischen Ring bilden können. |

2. Verfahren zur Bekämpfung von Insekten und Milben, dadurch gekennzeichnet, daß man substituierte $\alpha,\omega$-Aminoalkohol-Derivate der Formel (I) auf Insekten und/oder Milben und/oder ihren Lebensraum einwirken läßt.

3. Verwendung von substituierten $\alpha,\omega$-Aminoalkohol-Derivaten der Formel (I) zur Bekämpfung von Insekten und/oder Milben.

4. Verfahren zur Herstellung von Insekten- und milbenabwehrenden Mitteln, dadurch gekennzeichnet, daß man substituierte $\alpha,\omega$-Aminoalkohol-Derivate gemäß Formel (I) mit Streckmitteln und/oder oberflächenaktiven Mitteln vermischt.

5. Substituierte $\alpha,\omega$-Aminoalkohol-Derivate der Formel (VII)

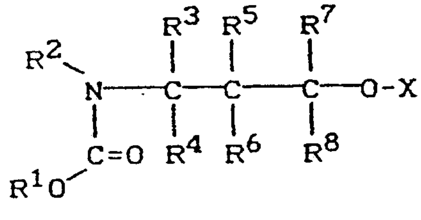

(VII)

in welcher

| | |
|---|---|
| X | für Wasserstoff, $COR^{11}$ oder $R^{13}$ steht, |
| $R^1$ | für $C_1$-$C_7$-Alkyl, $C_3$-$C_7$-Alkenyl oder $C_2$-$C_7$-Alkinyl steht, |
| $R^2$, $R^{11}$ und $R^{13}$ | gleich oder verschieden sind und für $C_1$-$C_6$-Alkyl oder $C_2$-$C_7$-Alkenyl stehen, |
| $R^3$-$R^8$ | gleich oder verschieden sind und für Wasserstoff oder $C_1$-$C_6$-Alkyl stehen, wobei $R^2$ und $R^3$ oder $R^3$ und $R^7$ oder $R^3$ und $R^5$ oder $R^5$ und $R^7$ auch gemeinsam mit den Atomen, an welche sie gebunden sind, einen 5- oder 6-gliedrigen monocyclischen Ring bilden können, |

mit Ausnahme folgender Substituentenkombinationen a) bis f):

a) X = Wasserstoff, $R^2$ = Methyl und $R^1$ = tert. Butyl,
b) X = Wasserstoff, $R^1$ = Ethyl, $R^5$ = Ethyl, $R^6$ = Ethyl,
c) X, $R^3$, $R^4$, $R^7$, $R^8$ = Wasserstoff, $R^1$, $R^5$, $R^6$ = Ethyl, $R^2$ = Methyl,
d) X, $R^3$, $R^4$, $R^8$ = Wasserstoff, $R^1$, $R^2$, $R^5$, $R^6$ = Ethyl, $R^7$ = Methyl,
e) X, $R^3$, $R^4$, $R^7$, $R^8$ = Wasserstoff, $R^1$, $R^2$, $R^5$, $R^6$ = Ethyl,
f) X, $R^3$, $R^4$, $R^7$, $R^8$ = Wasserstoff, $R^1$, $R^5$, $R^6$ = Ethyl, $R^2$ = n-Propyl,

**6.** Substituierte $\alpha,\omega$-Aminoalkohol-Derivate der Formel VII gemäß Anspruch 5,

in welcher

| | |
|---|---|
| X | für Wasserstoff oder $R^{13}$ steht, wobei $R^{13}$ für $C_1$-$C_6$-Alkyl steht, |
| $R^1$ | für $C_1$-$C_7$-Alkyl oder $C_3$-$C_7$-Alkenyl steht, |
| $R^4$ bis $R^8$ | gleich oder verschieden sind und für Wasserstoff oder $C_1$-$C_6$-Alkyl stehen, |
| $R^2$ und $R^3$ | gemeinsam mit den Atomen, an welche sie gebunden sind, einen 5- oder 6-gliedrigen monocyclischen Ring bilden. |

**7.** Substituierte $\alpha,\omega$-Aminoalkohol-Derivate der Formel VII gemäß Anspruch 5,

in welcher

| | |
|---|---|
| $R^1$ | für $C_1$-$C_7$-Alkyl, $C_3$-$C_7$-Alkenyl oder $C_2$-$C_7$-Alkinyl steht, |
| X | für Wasserstoff, $COR^{11}$ oder $R^{13}$ steht, |
| $R^2$ und $R^{11}$ | gleich oder verschieden sind und für $C_1$-$C_6$-Alkyl stehen, |
| $R^3$ bis $R^8$ | gleich oder verschieden sind und für Wasserstoff oder $C_1$-$C_6$-Alkyl stehen, |
| $R^{13}$ | für $C_1$-$C_6$-Alkyl steht, |

mit Ausnahme folgender Substituentenkombinationen a) bis f)

a) X = Wasserstoff, $R^2$ = Methyl und $R^1$ = tert. Butyl,
b) X = Wasserstoff, $R^1$, $R^5$ und $R^6$ = Ethyl.
c) X, $R^3$, $R^4$, $R^7$, $R^8$ = Wasserstoff, $R^1$, $R^5$, $R^6$ = Ethyl, $R^2$ = Methyl,
d) X, $R^3$, $R^4$, $R^8$ = Wasserstoff, $R^1$, $R^2$, $R^5$, $R^6$ = Ethyl, $R^7$ = Methyl,
e) X, $R^3$, $R^4$, $R^7$, $R^8$ = Wasserstoff, $R^1$, $R^2$, $R^5$, $R^6$ = Ethyl,
f) X, $R^3$, $R^4$, $R^7$, $R^8$ = Wasserstoff, $R^1$, $R^5$, $R^6$ = Ethyl, $R^2$ = n-Propyl,

**8.** Verfahren zur Herstellung von substituierten $\alpha,\omega$-Aminoalkohol-Derivaten der allgemeinen Formel VII

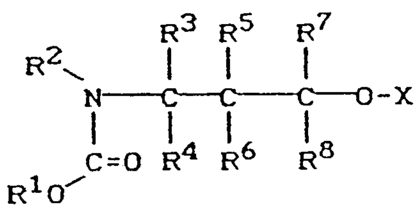

(VII)

in welcher

| | |
|---|---|
| X | für Wasserstoff, $COR^{11}$ oder $R^{13}$ steht, |
| $R^1$ | für $C_1$-$C_7$-Alkyl, $C_3$-$C_7$-Alkenyl oder $C_2$-$C_7$-Alkinyl steht, |
| $R^2$, $R^{11}$ und $R^{13}$ | gleich oder verschieden sind und für $C_1$-$C_6$-Alkyl oder $C_2$-$C_7$-Alkenyl stehen, |
| $R^3$-$R^8$ | gleich oder verschieden sind und für Wasserstoff oder $C_1$-$C_6$-Alkyl stehen, wobei $R^2$ und $R^3$ oder $R^3$ und $R^7$ oder $R^3$ und $R^5$ oder $R^5$ und $R^7$ auch gemeinsam mit den Atomen, an welche sie gebunden sind, einen 5- oder 6-gliedrigen monocyclischen Ring bilden können, |

mit Ausnahme folgender Substituentenkombinationen a) bis f):

a) X = Wasserstoff $R^2$ = Methyl und $R^1$ = tert. Butyl
b) X = Wasserstoff, $R^1$ = Ethyl, $R^5$ = Ethyl, $R^6$ = Ethyl.
c) X, $R^3$, $R^4$, $R^7$, $R^8$ = Wasserstoff, $R^1$, $R^5$, $R^6$ = Ethyl, $R^2$ = Methyl,
d) X, $R^3$, $R^4$, $R^8$ = Wasserstoff, $R^1$, $R^2$, $R^5$, $R^6$ = Ethyl, $R^7$ = Methyl,
e) X, $R^3$, $R^4$, $R^7$, $R^8$ = Wasserstoff, $R^1$, $R^2$, $R^5$, $R^6$ = Ethyl,
f) X, $R^3$, $R^4$, $R^7$, $R^8$ = Wasserstoff, $R^1$, $R^5$, $R^6$ = Ethyl, $R^2$ = n-Propyl,

dadurch gekennzeichnet, daß man

a) α,ω-Aminoalkohole der Formel (VIII)

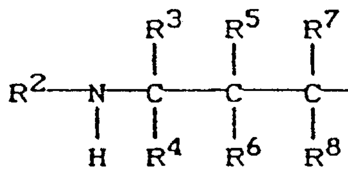

(VIII)

worin

$R^2$ bis $R^8$ die unter Formel (VII) angegebene Bedeutung besitzen,
mit Kohlensäurederivaten der Formel (IX)

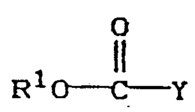

(IX)

wobei $R^1$ die unter Formel VII angegebene Bedeutung besitzt und Y für Halogen oder eine bei Amidierungsreaktionen übliche Abgangsgruppe steht,
gegebenenfalls in Gegenwart eines Verdünnungsmitteils und gegebenenfalls unter Zusatz einer Base, umsetzt,
die dabei erhaltenen Verbindungen der Formel (VII), in welcher X für Wasserstoff steht, gegebenenfalls isoliert
und gegebenenfalls zum Erhalt von Verbindungen der Formel (VII), in welcher X für $COR^{11}$ steht, mit Carbonsäurechloriden der Formel (IV)

$R^{11}$ - COCl (IV)

umsetzt,
oder gegebenenfalls zum Erhalt von Verbindungen der Formel (VII), in welcher X für $R^{13}$ steht, mit Alkylhalogeniden der Formel (VI)

$R^{13}$ - Y (VI)

worin Y für Chlor, Brom oder Iod steht, umsetzt,
b) oder daß man α,ω-Aminoalkohole bzw. α,ω-Aminoether der Formel (XII)

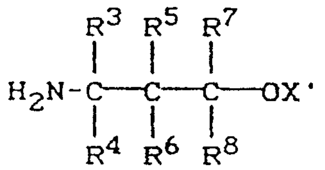

(XII)

worin $R^3$ bis $R^8$ die unter Formel (VII) angegebene Bedeutung besitzen, und worin X' für Wasserstoff oder $R^{13}$ steht, wobei $R^{13}$ für $C_1$-$C_6$-Alkyl steht, zunächst mit Chlorkohlensäureestern der Formel (III)

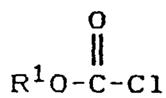

(III)

worin $R^1$ die unter Formel (VII) angegebene Bedeutung besitzt, gegebenenfalls in Gegenwart eines Säureakzeptors und gegebenenfalls unter Verwendung eines Verdünnungsmittels umsetzt, dann in einem zweiten Reaktionsschritt, gegebenenfalls nach Isolierung des Zwischenproduktes mit freier OH-Gruppe (X' = H),
zur Herstellung von Verbindungen der Formel (VII) mit X = $COR^{11}$,
wobei $R^{11}$ die obengenannte Bedeutung hat,
mit Carbonsäurechloriden der Formel (IV)

$R^{11}COCl$ (IV)

und nach gegebenenfalls erfolgter Isolierung des Zwischenproduktes mit freier NH-Gruppe, weiterhin mit Verbindungen der Formel (XI)

$R^2$-Y' (XI)

worin $R^2$ die oben angegebene Bedeutung hat und Y' für Chlor, Brom oder Iod steht, gegegebenenfalls in Gegenwart einer Base und gegebenenfalls unter Verwendung eines Verdünnungsmittels umsetzt.

## Claims

1. Agents for repelling insects and mites, characterised in that they contain at least one substituted $\alpha,\omega$-aminoalcohol derivative of the formula (I)

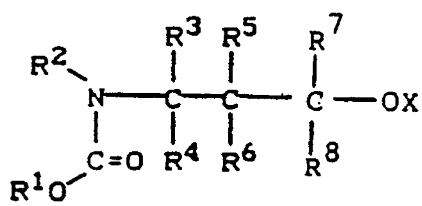

(I)

in which

| | |
|---|---|
| X | represents hydrogen, $COR^{11}$ or $R^{13}$, |
| $R^1$ | represents $C_1$-$C_7$-alkyl, $C_3$-$C_7$-alkenyl or $C_2$-$C_7$-alkinyl, |
| $R^2$, $R^{11}$ and $R^{13}$ | are identical or different and represent $C_1$-$C_6$-alkyl or $C_2$-$C_7$-alkenyl, |
| $R^3$-$R^8$ | are identical or different and represent hydrogen or $C_1$-$C_6$-alkyl, and wherein $R^2$ and $R^3$ or $R^3$ and $R^7$ or $R^3$ and $R^5$ or $R^5$ and $R^7$, together with the atoms to which they are bonded, can also form a 5- or 6-membered monocyclic ring. |

2. Method for combating insects and mites, characterised in that substituted α,ω-aminoalcohol derivatives of the formula (I) are allowed to act on insects and/or mites and/or their environment.

3. Use of substituted α,ω-aminoalcohol derivatives of the formula (I) for combating insects and/or mites.

4. Process for the preparation of agents for repelling insects and mites, characterised in that substituted α,ω-aminoalcohol derivatives according to formula (I) are mixed with extenders and/or surface-active agents.

5. Substituted α,ω-aminoalcohol derivatives of the formula (VII)

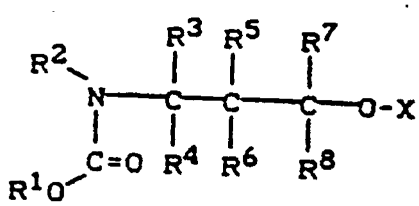

(VII)

in which

| | |
|---|---|
| X | represents hydrogen, $COR^{11}$ or $R^{13}$, |
| $R^1$ | represents $C_1$-$C_7$-alkyl, $C_3$-$C_7$-alkenyl or $C_2$-$C_7$-alkinyl, |
| $R^2$, $R^{11}$ and $R^{13}$ | are identical or different and represent $C_1$-$C_6$-alkyl or $C_2$-$C_7$-alkenyl, |
| $R^3$-$R^8$ | are identical or different and represent hydrogen or $C_1$-$C_6$-alkyl, and wherein $R^2$ and $R^3$ or $R^3$ and $R^7$ or $R^3$ and $R^5$ or $R^5$ and $R^7$, together with the atoms to which they are bonded, can also form a 5- or 6-membered monocyclic ring, |

with the exception of the following substituent combinations a) to f):

a) X = hydrogen, $R^2$ = methyl and $R^1$ = tert.-butyl,
b) X = hydrogen, $R^1$ = ethyl, $R^5$ = ethyl and $R^6$ = ethyl,
c) X, $R^3$, $R^4$, $R^7$ and $R^8$ = hydrogen, $R^1$, $R^5$ and $R^6$ = ethyl and $R^2$ = methyl,
d) X, $R^3$, $R^4$ and $R^8$ = hydrogen, $R^1$, $R^2$, $R^5$ and $R^6$ = ethyl and $R^7$ = methyl,
e) X, $R^3$, $R^4$, $R^7$ and $R^8$ = hydrogen and $R^1$, $R^2$, $R^5$ and $R^6$ = ethyl,
f) X, $R^3$, $R^4$, $R^7$ and $R^8$ = hydrogen, $R^1$, $R^5$ and $R^6$ = ethyl and $R^2$ = n-propyl.

6. Substituted α,ω-aminoalcohol derivatives of the formula VII according to Claim 5

in which

| | |
|---|---|
| X | represents hydrogen or $R^{13}$, wherein $R^{13}$ represents $C_1$-$C_6$-alkyl, |
| $R^1$ | represents $C_1$-$C_7$-alkyl or $C_3$-$C_7$-alkenyl, |
| $R^4$ to $R^8$ | are identical or different and represent hydrogen or $C_1$-$C_6$-alkyl, and |
| $R^2$ and $R^3$, | together with the atoms to which they are bonded, form a 5- or 6-membered monocyclic ring. |

7. Substituted α,ω-aminoalcohol derivatives of the formula VII according to Claim 5

in which

| | |
|---|---|
| $R^1$ | represents $C_1$-$C_7$-alkyl, $C_3$-$C_7$-alkenyl or $C_2$-$C_7$-alkinyl, |
| X | represents hydrogen, $COR^{11}$ or $R^{13}$, |
| $R^2$ and $R^{11}$ | are identical or different and represent $C_1$-$C_6$-alkyl, |
| $R^3$ to $R^8$ | are identical or different and represent hydrogen or $C_1$-$C_6$-alkyl, and |
| $R^{13}$ | represents $C_1$-$C_6$-alkyl, |

with the exception of the following substituent combinations a) to f)

a) X = hydrogen, $R^2$ = methyl and $R^1$ = tert.-butyl,
b) X = hydrogen and $R^1$, $R^5$ and $R^6$ = ethyl,
c) X, $R^3$, $R^4$, $R^7$ and $R^8$ = hydrogen, $R^1$, $R^5$ and $R^6$ = ethyl and $R^2$ = methyl,
d) X, $R^3$, $R^4$ and $R^8$ = hydrogen, $R^1$, $R^2$, $R^5$ and $R^6$ = ethyl and $R^7$ = methyl,
e) X, $R^3$, $R^4$, $R^7$ and $R^8$ = hydrogen and $R^1$, $R^2$, $R^5$ and $R^6$ = ethyl,
f) X, $R^3$, $R^4$, $R^7$ and $R^8$ = hydrogen, $R^1$, $R^5$ and $R^6$ = ethyl and $R^2$ = n-propyl.

**8.** Process for the preparation of substituted $\alpha,\omega$-aminoalcohol derivatives of the general formula VII

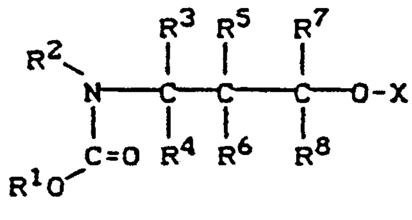

(VII)

in which

| | |
|---|---|
| X | represents hydrogen, $COR^{11}$ or $R^{13}$, |
| $R^1$ | represents $C_1$-$C_7$-alkyl, $C_3$-$C_7$-alkenyl or $C_2$-$C_7$-alkinyl, |
| $R^2$, $R^{11}$ and $R^{13}$ | are identical or different and represent $C_1$-$C_6$-alkyl or $C_2$-$C_7$-alkenyl, |
| $R^3$-$R^8$ | are identical or different and represent hydrogen or $C_1$-$C_6$-alkyl, and wherein $R^2$ and $R^3$ or $R^3$ and $R^7$ or $R^3$ and $R^5$ or $R^5$ and $R^7$, together with the atoms to which they are bonded, can also form a 5- or 6-membered monocyclic ring, |

with the exception of the following substituent combinations a) to f):

a) X = hydrogen, $R^2$ = methyl and $R^1$ = tert.-butyl,
b) X = hydrogen, $R^1$ = ethyl, $R^5$ = ethyl and $R^6$ = ethyl,
c) X, $R^3$, $R^4$, $R^7$ and $R^8$ = hydrogen, $R^1$, $R^5$ and $R^6$ = ethyl and $R^2$ = methyl,
d) X, $R^3$, $R^4$ and $R^8$ = hydrogen, $R^1$, $R^2$, $R^5$ and $R^6$ = ethyl and $R^7$ = methyl,
e) X, $R^3$, $R^4$, $R^7$ and $R^8$ = hydrogen and $R^1$, $R^2$, $R^5$ and $R^6$ = ethyl,
f) X, $R^3$, $R^4$, $R^7$ and $R^8$ = hydrogen, $R^1$, $R^5$ and $R^5$ = ethyl and $R^2$ = n-propyl, characterised in that

a) $\alpha,\omega$-aminoalcohols of the formula (VIII)

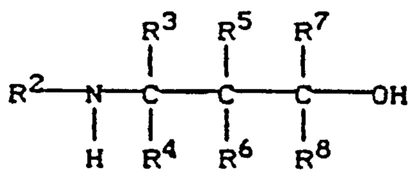

(VIII)

wherein
$R^2$ to $R^8$ have the meaning given under formula (VII),
are reacted with carboxylic acid derivatives of the formula (IX)

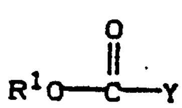

(IX)

wherein $R^1$ has the meaning given under formula VII and Y represents halogen or a leaving group customary for amidation reactions,

if appropriate in the presence of a diluent and if appropriate with the addition of a base,
if appropriate the compounds thereby obtained, of the formula (VII) in which X represents hydrogen, are isolated
and if appropriate, to obtain compounds of the formula (VII) in which X represents $COR^{11}$, the products are reacted with carboxylic acid chlorides of the formula (IV)

$R^{11}$-COCl (IV)

or if appropriate, to obtain compounds of the formula (VII) in which X represents $R^{13}$, the products are reacted with alkyl halides of the formula (VI)

$R^{13}$ - Y (VI)

wherein Y represents chlorine, bromine or iodine,
b) or in that
α,ω-aminoalcohols or α,ω-aminoethers of the formula (XII)

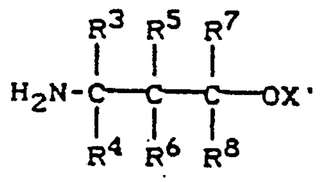

(XII)

wherein $R^3$ to $R^8$ have the meaning given under formula (VII),
and wherein X' represents hydrogen or $R^{13}$, wherein $R^{13}$ represents $C_1$-$C_6$-alkyl,
are first reacted with chlorocarbonic acid esters of the formula (III)

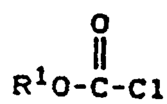

(III)

wherein $R^1$ has the meaning given under formula (VII), if appropriate in the presence of an acid acceptor and if appropriate using a diluent, and, in a second reaction step, if appropriate after isolation of the intermediate with the free OH group (X' = H),
to prepare compounds of the formula (VII) where X = $COR^{11}$,
wherein $R^{11}$ has the abovementioned meaning,
the products are reacted with carboxylic acid chlorides of the formula (IV)

$R^{11}$-COCl (IV)

and after isolation, if appropriate, of the intermediate with the free NH group,
the products are furthermore reacted with compounds of the formula (XI)

$R^2$-Y' (XI)

wherein
$R^2$ has the abovementioned meaning and
Y' represents chlorine, bromine or iodine,
if appropriate in the presence of a base and if appropriate using a diluent.

**Revendications**

1. Compositions de protection contre des insectes et des acariens, caractérisées par une teneur en au moins un dérivé substitué d'α,ω-amino-alcool de formule (I)

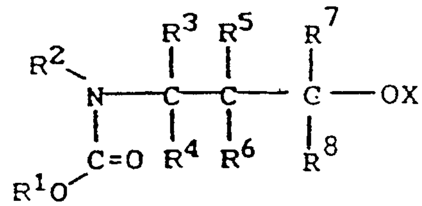

(I)

dans laquelle

| | |
|---|---|
| X | représente l'hydrogène, un groupe $COR^{11}$ ou $R^{13}$, |
| $R^1$ | est un groupe alkyle en $C_1$ à $C_7$, alcényle en $C_3$ à $C_7$ ou alcynyle en $C_2$ à $C_7$, |
| $R^2$, $R^{11}$, $R^{13}$ | sont identiques ou différents et représentent un groupe alkyle en $C_1$ à $C_6$ ou un groupe alcényle en $C_2$ à $C_7$, |
| $R^3$-$R^8$ | sont identiques ou différents et représentent l'hydrogène ou un groupe alkyle en $C_1$ à $C_6$, $R^2$ et $R^3$ ou $R^3$ et $R^7$ ou $R^3$ et $R^5$ ou $R^5$ et $R^7$ pouvant aussi former un noyau monocyclique pentagonal ou hexagonal conjointement avec l'atome auquel ils sont liés. |

**2.** Procédé pour combattre des insectes et des acariens, caractérisé en ce qu'on fait agir des dérivés substitués d'$\alpha,\omega$-amino-alcools de formule (I) sur des insectes et/ou des acariens et/ou sur leur milieu.

**3.** Utilisation de dérivés substitués d'$\alpha,\omega$-amino-alcools de formule (I) pour combattre des insectes et/ou des acariens.

**4.** Procédé de préparation de compositions permettant de se protéger contre des insectes et des acariens, caractérisé en ce qu'on mélange des dérivés substitués d'$\alpha,\omega$-amino-alcools de formule (I) avec des diluants et/ou des agents tensio-actifs.

**5.** Dérivés substitués d'$\alpha,\omega$-amino-alcools de formule (VII)

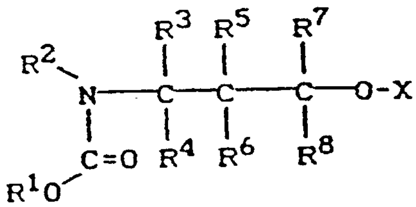

(VII)

dans laquelle

| | |
|---|---|
| X | représente l'hydrogène, un groupe $COR^{11}$ ou $R^{13}$, |
| $R^1$ | est un groupe alkyle en $C_1$ à $C_7$, alcényle en $C_3$ à $C_7$ ou alcynyle en $C_2$ à $C_7$, |
| $R^2$, $R^{11}$ et $R^{13}$ | sont identiques ou différents et représentent un groupe alkyle en $C_1$ à $C_6$ ou alcényle en $C_2$ à $C_7$, |
| $R^3$-$R^8$ | sont identiques ou différents et représentent l'hydrogène ou un groupe alkyle en $C_1$ à $C_6$, $R^2$ et $R^3$ ou $R^3$ et $R^7$ ou $R^3$ et $R^5$ ou $R^5$ et $R^7$ pouvant aussi former un noyau monocyclique pentagonal ou hexagonal conjointement avec les atomes auxquels ils sont liés, |

à l'exception des associations de substituants a) à f) suivantes :

a) X = hydrogène, $R^2$ = méthyle et $R^1$ = tertiobutyle,
b) X = hydrogène, $R^1$ = éthyle, $R^5$ = éthyle, $R^6$ = éthyle,
c) X, $R^3$, $R^4$, $R^7$, $R^8$ = hydrogène, $R^1$, $R^5$, $R^6$ = éthyle, $R^2$ = méthyle,
d) X, $R^3$, $R^4$, $R^8$ = hydrogène, $R^1$, $R^2$, $R^5$, $R^6$ = éthyle, $R^7$ = méthyle,
e) X, $R^3$, $R^4$, $R^7$, $R^8$ = hydrogène, $R^1$, $R^2$, $R^5$, $R^6$ = éthyle,
f) X, $R^3$, $R^4$, $R^7$, $R^8$ = hydrogène, $R^1$, $R^5$, $R^6$ = éthyle, $R^2$ = n-propyle.

6. Dérivés substitués d'$\alpha,\omega$-amino-alcools de formule VII suivant la revendication 5, dans laquelle

| | |
|---|---|
| X | représente l'hydrogène ou $R^{13}$, $R^{13}$ étant un groupe alkyle en $C_1$ à $C_6$, |
| $R^1$ | est un groupe alkyle en $C_1$ à $C_7$ ou alcényle en $C_3$ à $C_7$, |
| $R^4$ à $R^8$ | sont identiques ou différents et représentent l'hydrogène ou un groupe alkyle en $C_1$ à $C_6$, |
| $R^2$ et $R^3$ | forment un noyau monocyclique pentagonal ou hexagonal conjointement avec les atomes auxquels ils sont liés. |

7. Dérivés substitués d'$\alpha,\omega$-amino-alcools de formule VII suivant la revendication 5, dans laquelle

| | |
|---|---|
| $R^1$ | est un groupe alkyle en $C_1$ à $C_7$, alcényle en $C_3$ à $C_7$ ou alcynyle en $C_2$ à $C_7$, |
| X | représente l'hydrogène, un groupe $COR^{11}$ ou $R^{13}$, |
| $R^2$ et $R^{11}$ | sont identiques ou différents et représentent un groupe alkyle en $C_1$ à $C_6$, |
| $R^3$ à $R^8$ | sont identiques ou différents et représentent l'hydrogène ou un groupe alkyle en $C_1$ à $C_6$, |
| $R^{13}$ | est un groupe alkyle en $C_1$ à $C_6$, |

à l'exception des associations de substituants a) à f) suivantes

a) X = hydrogène, $R^2$ = méthyle et $R^1$ = tertiobutyle,
b) X = hydrogène, $R^1$, $R^5$ et $R^6$ = éthyle,
c) X, $R^3$, $R^4$, $R^7$, $R^8$ = hydrogène, $R^1$, $R^5$, $R^6$ = éthyle, $R^2$ = méthyle,
d) X, $R^3$, $R^4$, $R^8$ = hydrogène, $R^1$, $R^2$, $R^5$, $R^6$ = éthyle, $R^7$ = méthyle,
e) X, $R^3$, $R^4$, $R^7$, $R^8$ = hydrogène, $R^1$, $R^2$, $R^5$, $R^6$ = éthyle,
f) X, $R^3$, $R^4$, $R^7$, $R^8$ = hydrogène, $R^1$, $R^5$, $R^6$ = éthyle, $R^2$ = n-propyle.

8. Procédé de préparation de dérivés substitués d'$\alpha,\omega$-amino-alcools de formule générale VII

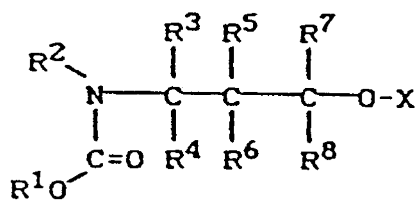

(VII)

dans laquelle

| | |
|---|---|
| X | représente l'hydrogène, un groupe $COR^{11}$ ou $R^{13}$, |
| $R^1$ | est un groupe alkyle en $C_1$ à $C_7$, alcényle en $C_3$ à $C_7$ ou alcynyle en $C_2$ à $C_7$, |
| $R^2$, $R^{11}$ et $R^{13}$ | sont identiques ou différents et représentent un groupe alkyle en $C_1$ à $C_6$ ou alcényle en $C_2$ à $C_7$, |
| $R^3$-$R^8$ | sont identiques ou différents et représentent l'hydrogène ou un groupe alkyle en $C_1$ à $C_6$, $R^2$ et $R^3$ ou $R^3$ et $R^7$ ou $R^3$ et $R^5$ ou $R^5$ et $R^7$ pouvant aussi former un noyau monocyclique pentagonal ou hexagonal conjointement avec les atomes auxquels ils sont liés, |

à l'exception des associations de substituants a) à f) suivantes :

a) X = hydrogène, $R^2$ = méthyle et $R^1$ = tertiobutyle,
b) X = hydrogène, $R^1$ = éthyle, $R^5$ = éthyle, $R^6$ = éthyle,
c) X, $R^3$, $R^4$, $R^7$, $R^8$ = hydrogène, $R^1$, $R^5$, $R^6$ = éthyle, $R^2$ = méthyle,
d) X, $R^3$, $R^4$, $R^8$ = hydrogène, $R^1$, $R^2$, $R^5$, $R^6$ = éthyle, $R^7$ = méthyle,
e) X, $R^3$, $R^4$, $R^7$, $R^8$ = hydrogène, $R^1$, $R^2$, $R^5$, $R^6$ = éthyle,
f) X, $R^3$, $R^4$, $R^7$, $R^8$ = hydrogène, $R^1$, $R^5$, $R^6$ = éthyle, $R^2$ = n-propyle,

caractérisé en ce que

a) on fait réagir des $\alpha,\omega$-amino-alcools de formule (VIII)

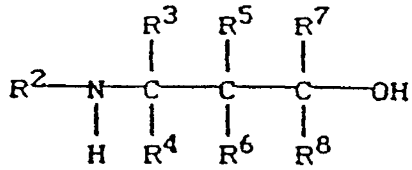

(VIII)

dans laquelle

$R^2$ à $R^8$ ont la définition indiquée pour la formule (VII)

avec des dérivés d'acide carbonique de formule (IX)

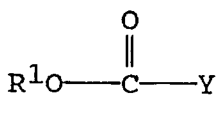

(IX)

dans laquelle $R^1$ a la définition indiquée pour la formule VII et Y est un halogène ou un groupe partant classique dans des réactions d'amidation,

le cas échéant en présence d'un diluant et avec addition éventuelle d'une base,

on isole éventuellement les composés ainsi obtenus de formule (VII), dans laquelle X est l'hydrogène,

et on les fait réagir, le cas échéant, pour obtenir des composés de formule (VII) dans laquelle X représente $COR^{11}$, avec des chlorures d'acides carboxyliques de formule (IV)

$R^{11}$ - COCl (IV)

où bien, le cas échéant, pour obtenir des composés de formule (VII) dans laquelle X représente $R^{13}$, on les fait réagir avec des halogénures d'alkyle de formule (VI)

$R^{13}$ - Y (VI)

dans laquelle Y représente le chlore, le brome ou l'iode, ou bien

b) on fait réagir des $\alpha,\omega$-amino-alcools ou des $\alpha,\omega$-amino-éthers de formule (XII)

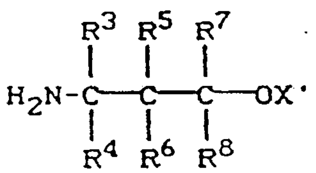

(XII)

dans laquelle $R^3$ à $R^8$ ont la définition indiquée pour la formule (VII),

et X' représente l'hydrogène ou $R^{13}$, $R^{13}$ étant un groupe alkyle en $C_1$ à $C_6$,

tout d'abord avec des esters d'acide chlorocarbonique de formule (III)

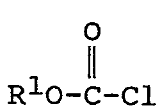

(III)

dans laquelle $R^1$ a la définition indiquée pour la formule (VII), le cas échéant en présence d'un accepteur d'acide et en utilisant éventuellement un diluant, puis dans une seconde étape réactionnelle, le cas échéant après isolement du produit intermédiaire portant un groupe OH libre (X' = H), pour l'obtention de composés de formule (VII) dans laquelle X représente $COR^{11}$, $R^{11}$ ayant la

définition indiquée ci-dessus, on les fait réagir avec des chlorures d'acides carboxyliques de formule (IV)

$R^{11}COCl$ (IV)

et après que l'isolement du produit intermédiaire portant un groupe NH libre a été éventuellement effectué,
on les fait réagir en outre avec des composés de formule (XI)

$R^2$-Y' (XI)

dans laquelle $R^2$ a la définition indiquée ci-dessus et Y' représente le chlore, le brome ou l'iode, éventuellement en présence d'une base et en utilisant, le cas échéant, un diluant.